# EUROPEAN PATENT APPLICATION

(11) **EP 4 603 598 A1**
(43) Date of publication of application: **20.08.2025**
(21) Application number: 24157360.9
(22) Date of filing: 13.02.2024
(51) Int. Cl.: C12Q 1/6881, C12Q 1/6886

(54) **IDENTIFICATION OF REACTIVE T CELLS**

(71) Applicant: Deutsches Krebsforschungszentrum Stiftung des öffentlichen Rechts, 69120 Heidelberg (DE); Universität Heidelberg, 69117 Heidelberg (DE)
(72) Inventor: GREEN, Edward, 69120 Heidelberg (DE); TAN, Chin Leng, 69120 Heidelberg (DE); PLATTEN, Michael, 69120 Heidelberg (DE)
(74) Representative: Altmann Stößel Dick Patentanwälte PartG mbB

(57) **Abstract**

The present invention relates to a method of identifying a T cell reactive to target cells of a subject presenting a T cell activating epitope (reactive T cell), comprising (a) determining expression of at least one biomarker selected from the group consisting of MAGEH1, HMGB2, TMSB10, MGAT4A, C12orf57, GIMAP7, HSPE1, STMN1, IL32, TPT1, MIF, PALM2-AKAP2, ISCU, CCR8, UGP2, EEF1B2, SETD7, MESD, LINC02099, IFT52, AKIRIN2, RIOK3, ANKRD12, CALR, DDX5, CNST, GNPDA1, MALAT1, RGL4, B2M, IFITM2, PTPN11, BTG1, ITSN2, RAD21, FYB1, DDX21, HSPD1, JAK1, ADAM19, TM2D3, CASP8, CARS1, PTPN7, HAPLN3, CSTB, TLE5, ACTG2, IL2RA, CD99, TMSB4X, CCR4, and PCM1 in T cells from a sample of said subject; and (b) identifying a reactive T cell based on the determination of step (a). The present invention also relates to further methods, reactive T cells, and polynucleotides related thereto.

## Description

The present invention relates to a method of identifying a T cell reactive to target cells of a subject presenting a T cell activating epitope (reactive T cell), comprising (a) determining expression of at least one biomarker selected from the group consisting of MAGEH1, HMGB2, TMSB10, MGAT4A, C12orf57, GIMAP7, HSPE1, STMN1, IL32, TPT1, MIF, PALM2-AKAP2, ISCU, CCR8, UGP2, EEF1B2, SETD7, MESD, LINC02099, IFT52, AKIRIN2, RIOK3, ANKRD12, CALR, DDX5, CNST, GNPDA1, MALAT1, RGL4, B2M, IFITM2, PTPN11, BTG1, ITSN2, RAD21, FYB1, DDX21, HSPD1, JAK1, ADAM19, TM2D3, CASP8, CARS1, PTPN7, HAPLN3, CSTB, TLE5, ACTG2, IL2RA, CD99, TMSB4X, CCR4, and PCM1 in T cells from a sample of said subject; and (b) identifying a reactive T cell based on the determination of step (a). The present invention also relates to further methods, reactive T cells, and polynucleotides related thereto.

Over recent years, there has been increasing interest in identifying T cells reactive to disease-associated antigens and their T cell receptors (TCRs) for treatment of a variety of diseases, such as cancers. In such a therapy, reactive T cells may be administered as such, or T cells transgenically modified to express e.g. a tumor reactive TCR may be used.

As a source of T cells for identifying e.g. tumor-reactive TCRs, tumor-infiltrating lymphocytes (TILs) have been used. Tumor reactive T cells within a TIL population can in theory be identified by their upregulation of known T cell activation biomarkers such as CD69 and Nur77, although in practice the value of TCRs identified by such an approach has been found to be limited. Further biomarkers of T cell activation have been described, cf. e.g. Cano-Gamez et al., Nat Comm 11:, art. 1801 (2020) (doi.org/10.1038/s41467-020-15543-y), Magen et al., Cell Rep 29(10):3019 (2019), and Oh et al., Cell 181(7):1612 (2020); WO 2022/200456 A1; WO 2022/200457 A1. Moreover, e.g. biomarkers predicting non-response to immune checkpoint blockade (WO2018/209324) and biomarkers for immunotherapy resistance (WO2019/070755) have been described. Recently, activation markers from tumor infiltrating T lymphocytes were described (WO 2021/188954 A1, Lowery et al., Science 375, 877-884 (2022)).

The success of TIL therapy trials in metastatic melanoma shows that TILs contain a fraction of tumor-reactive T cells which can be harnessed for adoptive cell therapy (Rohaan et al., N. Engl. J. Med. 387, 2113-2125 (2022)), although this success is more limited in non-melanoma cancer types 2 where the baseline fraction of experimentally verifiable, tumor-reactive CD8+ T cells is low. While the fraction of tumor-reactive T cells can be enriched prior to reinfusion, this cell expansion process can exhaust the T cells, compromising their tumor killing efficacy (Crompton et al., Immunol. Rev. 257, 264-276 (2014)), and may lead to the loss of clones (Poschke et al., Clin. Cancer Res. 26, 4289-4301 (2020)). In contrast, personalized transgenic T cell therapies seek to identify and reinfuse defined tumor-reactive TCRs, either in patient autologous T cells or iPSC derived, hypoimmunogenic (allogeneic) T cells. While this generates a highly efficacious product, identifying tumor-reactive TCRs is a 'needle in a haystack' problem (Simoni et al., Nature 557, 575-579 (2018)). Biomarkers for identifying reactive T cells, presumed to carry reactive TCRs, were proposed e.g. for pancreatic cancer (Meng et al., Sci. Transl. Med. 15, eadh9562 (2023)), metastatic colorectal cancer (Lowery et al., Science 375, 877-884 (2022)), non-small cell lung cancer (Caushi. et al., Nature 596, 126-132 (2021)) and for gastrointestinal cancer (Zheng et al. Cancer Cell 40, 410-423.e7 (2022)).

There is nonetheless still a need for improved methods for providing T cells reactive to specific antigens, e.g. cancer antigens, and corresponding TCRs. This problem is solved by the embodiments characterized in the claims and described herein below.

In accordance, the present invention relates to a method of identifying a T cell reactive to target cells of a subject presenting a T cell activating epitope (reactive T cell), comprising
(a) determining expression of at least one biomarker selected from the group consisting of MAGEH1, HMGB2, TMSB10, MGAT4A, C12orf57, GIMAP7, HSPE1, STMN1, IL32, TPT1, MIF, PALM2-AKAP2, ISCU, CCR8, UGP2, EEF1B2, SETD7, MESD, LINC02099, IFT52, AKIRIN2, RIOK3, ANKRD12, CALR, DDX5, CNST, GNPDA1, MALAT1, RGL4, B2M, IFITM2, PTPN11, BTG1, ITSN2, RAD21, FYB1, DDX21, HSPD1, JAK1, ADAM19, TM2D3, CASP8, CARS1, PTPN7, HAPLN3, CSTB, TLE5, ACTG2, IL2RA, CD99, TMSB4X, CCR4, and PCM1 in T cells from a sample of said subject; and
(b) identifying a reactive T cell based on the determination of step (a).

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of (B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one". In accordance, expressions relating to one item of a plurality, unless otherwise indicated, preferably relate to at least one such item, more preferably a plurality thereof; thus, e.g. identifying "a cell" relates to identifying at least one cell, preferably to identifying a plurality of cells. The term "plurality" is used herein in its common meaning to relate to a quantity of more than one, i.e. at least two. Thus, a plurality may preferably be a number of two or more, three or more, five or more, ten or more, 25 or more, 50 or more 100 or more, 1000 or more.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein below, preferably, are in vitro methods, unless indicated otherwise. The method steps may, in principle, be performed in any arbitrary sequence deemed suitable by the skilled person, but preferably are performed in the indicated sequence; also, one or more, preferably all, of said steps may be assisted or performed by automated equipment. Moreover, the methods may comprise steps in addition to those explicitly mentioned above.

As used herein, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1 % by weight, most preferably less than 0.1% by weight of non-specified component(s).

Basic statistic tools are known in the art. Thus, whether e.g. a portion or a feature of a population is statistically significant can be determined without further ado by the person skilled in the art using various well known statistic evaluation tools, e.g., determination of confidence intervals, p-value determination, Student's t-test, Mann-Whitney test etc. Details are found in textbooks, such as Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.05, 0.01, 0.005, or 0.0001.

The term "polynucleotide", as used herein, refers to a linear or circular nucleic acid molecule. The polynucleotide of the present invention shall be provided, preferably, either as an isolated polynucleotide (i.e. isolated from its natural context) or in genetically modified form, preferably comprising at least one heterologous sequence. The term polynucleotide encompasses single-as well as, partially or completely, double-stranded polynucleotides. Preferably, the polynucleotide is a DNA polynucleotide, which may also be referred to as "DNA". Moreover, comprised are also chemically modified polynucleotides including naturally occurring modified polynucleotides such as glycosylated or methylated polynucleotides or artificially modified derivatives such as biotinylated polynucleotides, locked nucleic acids, peptide nucleic acids, and the like. In view of the description herein, template DNA, e.g. for PCR, primers, e.g. sequencing or amplification primers, and probe oligonucleotides are included in the term polynucleotides.

Unless specifically indicated otherwise, reference to specific polynucleotides herein preferably includes polynucleotide variants. The term "polynucleotide variant", as used herein, relates to a variant of a polynucleotide referred to herein comprising a nucleic acid sequence characterized in that the sequence can be derived from the aforementioned specific nucleic acid sequence by at least one nucleotide substitution, addition and/or deletion, wherein the polynucleotide variant shall have the function and/or activity as specified for the specific polynucleotide. Thus, a variant of a polynucleotide may e.g. be an ortholog, a paralog, or another homolog of the specific polynucleotide; the polynucleotide variant may also be a mutant of the specific polynucleotide, preferably a naturally occurring mutant, e.g. identified in a cancer cell. Also preferably, said polynucleotide variant is or is derived from a non-naturally occurring allele of the specific polynucleotide. Further polynucleotide variants include polynucleotides comprising nucleic acid sequences which are at least 70%, preferably at least 80%, more preferably at least 90%, even more preferably at least 95%, still more preferably at least 98%, most preferably at least 99%, identical to the specifically indicated nucleic acid sequences. The percent identity values are, preferably, calculated over the entire nucleic acid sequence region, preferably as specified herein elsewhere. The polynucleotides of the present invention either consist, essentially consist of, or comprise the aforementioned nucleic acid sequences. Thus, they may contain further nucleic acid sequences as well.

The term "protein" is understood by the skilled person; preferably, the protein comprises at least one amino acid chain, i.e. a polypeptide as specified herein below, more preferably comprises a plurality of polypeptides. Thus, the protein may be a multimer, e.g. a dimer, a trimer, or the like, wherein the polypeptides in the multimer may be connected covalently, e.g. by a disulfide bridge, or non-covalently, e.g. by ionic interactions, hydrophobic interactions, and/or van der Waals interactions. The protein may consist of identical polypeptides, e.g. may be a homodimer, or may comprise at least two nonidentical polypeptides, e.g. may be a heterodimer. More preferably, the protein as specified comprises all structural components as indicated comprised in one continuous covalent polypeptide chain, thus, the protein preferably is or is comprised in a fusion polypeptide.

The term "polypeptide", as used herein, refers to a molecule consisting of a plurality of amino acids that are covalently linked to each other by peptide bonds. Polypeptides consisting of less than 20 amino acids covalently linked by peptide bonds may also be referred to as "peptides". Preferably, the polypeptide comprises of from 100 to 1000, more preferably of from 150 to 1000, still more preferably of from 200 to 500, most preferably of from 250 to 400 amino acids. The polypeptide may also be comprised in a fusion polypeptide, i.e. may comprise amino acid sequences in addition to those specifically indicated. Also, the polypeptide may comprise additional, non-peptidic structures, such as at least one glycosylation, lipid conjugation, and the like. Thus, unless specifically indicated otherwise, reference to specific polypeptides herein preferably includes polypeptide variants.

As used herein, the term "polypeptide variant" relates to any chemical molecule comprising at least one polypeptide as specified herein differing in structure from a specifically indicated polypeptide. Preferably, the polypeptide variant comprises a polypeptide having a contiguous amino acid sequence corresponding to at least 70%, preferably at least 80%, more preferably at least 90%, even more preferably at least 95%, still more preferably at least 98%, most preferably at least 99%, of the amino acid sequence of the polypeptide specifically indicated. Moreover, a polypeptide variant as referred to herein may have an amino acid sequence which differs due to at least one amino acid substitution, deletion and/or addition, wherein the amino acid sequence of the variant is still, preferably, at least 70%, more preferably at least 80%, even more preferably at least 90%, even more preferably at least 95%, still more preferably at least 98%, most preferably at least 99%, identical with the amino acid sequence of the specific polypeptide. The degree of identity between two amino acid sequences can be determined by algorithms well known in the art and as described herein elsewhere. Polypeptide variants referred to above may be allelic variants or any other species specific homologs, paralogs, or orthologs. Moreover, the polypeptide variants referred to herein include fragments of the specific polypeptides or the aforementioned types of polypeptide variants, preferably as long as these fragments and/or variants have the activity as specified. Such fragments may be or may be derived from, e.g., degradation products or splice variants of the polypeptides. Further included are variants which differ due to posttranslational modifications such as phosphorylation, glycosylation, ubiquitinylation, sumoylation, or myristylation, by including non-natural amino acids, and/or by being peptidomimetics. The "activity" of a polypeptide as specified herein is, preferably, preserved in the polypeptide variant; as the skilled person will understand in view of the description herein, the activity of a polypeptide does not necessarily have to reflect its main natural function, but may be a further activity relevant in the context of the claimed invention. The above applies to peptide variants and to protein variants mutatis mutandis.

The term "fragment" of a biological macromolecule, preferably of a polynucleotide or polypeptide, is used herein in a wide sense relating to any sub-part, preferably subdomain, of the respective biological macromolecule comprising the indicated sequence, structure and/or activity. Thus, the term includes sub-parts generated by actual fragmentation of a biological macromolecule, but also sub-parts derived from the respective biological macromolecule in an abstract manner, e.g. in silico. Thus, as used herein, an Fc or Fab fragment, but also e.g. a single-chain antibody, a bispecific antibody, and a nanobody may be referred to as fragments of an immunoglobulin. Also, a disease epitope may be a fragment of a disease antigen.

Unless specifically indicated otherwise herein, the compounds specified, in particular the polynucleotides and polypeptides, may be comprised in larger structures, e.g. may be covalently or non-covalently linked to further sequences, adjuvants, carrier molecules, retardants, and other excipients. In particular, polypeptides as specified may be comprised in fusion polypeptides comprising further polypeptides, which may serve e.g. as a tag for purification and/or detection, as a linker, or to extend the in vivo half-life of a compound. The term "detectable tag" refers to a stretch of amino acids which are added to or introduced into the fusion polypeptide; preferably, the tag is added C- or N- terminally to the fusion polypeptide. Said stretch of amino acids preferably allows for detection of the polypeptide by an antibody which specifically recognizes the tag; or it preferably allows for forming a functional conformation, such as a chelator; or it preferably allows for visualization, e.g. in the case of fluorescent tags. Preferred detectable tags are the Myc-tag, FLAG-tag, 6-His-tag, HA-tag, GST-tag or a fluorescent protein tag, e.g. a GFP-tag. These tags are all well known in the art. Other further peptides preferably comprised in a fusion polypeptide comprise further amino acids or other modifications which may serve as mediators of secretion, as mediators of blood-brain-barrier passage, as cell-penetrating peptides, and/or as immune stimulants. Further polypeptides or peptides to which the polypeptides may be fused are signal and/or transport sequences, e.g. an IL-2 signal sequence, and linker sequences.

The degree of identity (e.g. expressed as "%identity") between two biological sequences, preferably DNA, RNA, or amino acid sequences, can be determined by algorithms well known in the art. Preferably, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, preferably over the whole length of the polynucleotide or polypeptide as specified herein, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (e.g. BLAST, GAP, BESTFIT, PASTA, or TFASTA), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. More preferably, the Basic Local Alignment Search Tool (BLAST) implementation is used with default parameter values for alignment. In the context of biological sequences referred to herein, the term "essentially identical" indicates a %identity value of at least 90%, preferably at least 95%, more preferably at least 98%, most preferably at least 99%. As will be understood, the term essentially identical includes 100% identity. The aforesaid applies to the term "essentially complementary" mutatis mutandis.

The term "T cell" is understood by the skilled person to relate to a lymphocyte expressing at least one type of T cell receptor. Preferably, the T cell is a CD8+ T cell recognizing major histocompatibility (MHC) class I molecules on the surface of target cells, or is a CD4+ T cell recognizing MHC class II molecules on the surface of target cells. Preferably, the T cell is a cytotoxic T cell, more preferably a CD8+ cytotoxic T cell, which may also be referred to as "killer cell". Also preferably, the T cell is a regulatory or helper T cell, more preferably a regulatory T cell. Preferably, the T cell is reactive to a disease epitope, preferably a cancer epitope, i.e. is a cancer-reactive T cell. Thus, preferably, the T cell expresses a TCR recognizing a disease epitope. Preferably, the T cell is a recombinant cell expressing a chimeric epitope receptor (CAR) and/or a recombinant T cell receptor. Methods of producing appropriate recombinant T cells are known in the art. As is known to the skilled person, human MHCs may also be referred to as "human leukocyte antigens" (HLAs).

The term "complementarity determining region", abbreviated as "CDR", is understood by the skilled person. As is known in the art, each TCR alpha, beta, gamma, and delta chain comprises three CDRs, which comprise the amino acids essentially providing the contacts of a TCR to a peptide presented by an MHC molecule as specified elsewhere herein. As is known to the skilled person, in TCRs, epitope binding specificity is essentially determined by the variable domain and specifically by the CDR3 region of the variable region. Thus, to reproduce the binding properties of a given TCR, it may be sufficient to transfer the CDR3s of said TCR to a suitable backbone, e.g. of another TCR. More preferably, in such case, all CDRs 1 to 3, or the variable regions or fragments thereof, are transferred.

The term "T cell receptor", abbreviated as "TCR", as used herein, relates to a polypeptide complex on the surface of T cells mediating recognition of antigenic peptides presented by target cells, preferably in the context of MHC molecules or MHC-related molecules such as MR1 or CD1, more preferably in the context of MHC molecules, still more preferably in the context of MHC class I or MHC class II molecules, most preferably in the context of MHC class I molecules. Typically, the TCR comprises one TCR-alpha chain and one TCR-beta chain, i.e. is an alpha/beta chain heterodimer. The TCR may, however, also comprise a TCR gamma and a TCR delta chain instead of the TCR alpha and beta chains. The TCR alpha and beta or gamma and delta chains mediate antigen recognition and each comprise a transmembrane region, a constant region, a joining region, and a variable region, the variable region of each TCR alpha, beta, gamma, or delta chain comprising three complementarity determining regions (CDRs), referred to as CDR1, CDR2, and CDR3, respectively. In accordance with usual nomenclature, the complex consisting of an alpha and a beta chain or a gamma and a delta chain is referred to as "T cell receptor" or "TCR" herein, the alpha and/or beta chain and the gamma and/or delta chains commonly or singly being referred to a "TCR polypeptide" or "TCR polypeptides", whereas the polypeptide complex comprising a TCR and accessory polypeptides, such as CD3 and CD247, is referred to as "T cell receptor complex", abbreviated as "TCR complex". Preferably the T cell receptor binds to a major histocompatibility complex (MHC) molecule, preferably an MHC class I or class II, more preferably an MHC class I molecule, presenting an epitope contributing to and/or associated with disease, preferably a cancer epitope. Binding of a T cell receptor to an antigen can be determined by methods known to the skilled person, e.g. by a tetramer assay. Preferably, binding of the TCR to an epitope presented on an MHC activates the T cell. Activation biomarkers of various types of T cells are known in the art and include in particular CD107a, CD69, CD137, CD27, TRAP/CD40L, and CD134. The TCR may also be a soluble TCR. The term "soluble TCR" is, in principle, known to the skilled person to relate to a TCR as specified herein above lacking the transmembrane domains. Thus, preferably, the soluble TCR comprises the constant and the variable regions of the TCR polypeptides of a TCR. More preferably, the soluble TCR comprises the variable regions of the TCR polypeptides of a TCR, preferably in the form of a fusion polypeptide.

The term "antigen", in principle, includes each and every immunogenic chemical compound, i.e. each and every chemical compound having the activity to induce an immune response to its own structure in a subject. As the skilled person understands, immunogenicity of a given chemical compound to a given subject depends on a variety of factors, including the health state of the subject, the mode of administration of the antigen, and other factors. Also, in case of a cancer antigen, tumor micro-environment and/or immune suppression may decrease or prevent immunogenicity of an antigen. Thus, as referred to herein, an antigen preferably is a chemical compound inducing an immune response to its own structure in a statistically significant portion of subjects to which the antigen is administered, wherein said subjects preferably are apparently healthy subjects. Preferably, the antigen has the activity of inducing a T cell response in a subject. Preferably, the antigen has the activity of stimulating antigen specific T cells, the term "antigen specific T cells" relating to T cells presenting on their surface T cell receptor molecules specifically recognizing, i.e. binding to, an epitope of the antigen, i.e. preferably to a disease epitope of the present invention, presented in the context of an MHC molecule. Preferably, the antigen is a disease antigen, i.e. an antigen comprised in an agent causing disease as described herein above. Thus, the antigen may in particular be a cancer antigen. Preferably, the antigen is a biological macromolecule, more preferably is a polypeptide.

The term "cancer antigen" relates to an antigen, preferably a polypeptide, expressed by a cancer cell. Preferably, the cancer antigen is expressed at an at least 5fold, preferably at least 10fold, more preferably at least 25fold, lower rate in non-cancer cells compared to cancer cells. Preferably, the cancer antigen is not expressed in non-cancer cells of a subject, preferably of the same tissue, more preferably is not expressed in non-cancer cells of a subject; thus, the cancer antigen preferably is a cancer specific antigen. More preferably, the cancer antigen is a neoantigen and/or comprises a neoepitope, expressed by cancer cells. Preferably, one or more peptides of the cancer antigen are presented via MHC molecules, more preferably MHC class-I molecules, on the surface of cancer cells. As specified elsewhere herein, the cancer preferably is a solid cancer, i.e. a tumor-forming cancer; thus, the cancer antigen preferably is a tumor antigen, more preferably a tumor-specific antigen.

The term "epitope" is known to the skilled person to relate to a (sub-)structure of an antigen which is recognizable for an immune system of a subject, wherein the epitope may be recognizable as such, e.g. to an antibody, or may require accessory factors for recognition; e.g. a T cell epitope may be recognizable to the immune system when presented as an MHC-I or MHC-II complex. As referred to herein, the epitope preferably is a sequence of amino acids, preferably a contiguous sequence of amino acids, i.e. a peptide. Preferably, the epitope has a length of at least three, more preferably at least four, more preferably at least five, most preferably at least six amino acids. Also preferably, the epitope has a length of at most 50, more preferably at most 25, even more preferably at most 20, most preferably at most 15 amino acids. Thus, preferably, the epitope has a length of from 3 to 50, more preferably from 4 to 25, even more preferably of from 5 to 20, most preferably of from 6 to 15 amino acids. The epitope, in particular the cancer epitope, may be an individual epitope, i.e. identified in a sample of the subject to be treated; it may, however, also be a shared disease epitope, i.e. a disease epitope known or assumed to be shared by a plurality of subjects, e.g. by a plurality of cancers. Shared cancer antigens and corresponding epitopes are well-known in the art.

The epitope as referred to herein is recognized by T cells; thus, the epitope is a T cell epitope. The term "T cell epitope", as used herein, relates to a contiguous sequence of amino acids comprised in a polypeptide, which bind to a major histocompatibility complex (MHC) class I or class II molecule to be presented on the surface of a cell (MHC-I) or of a professional antigen presenting cell (MHC-II). The skilled artisan knows how to predict immunogenic peptides presented on MHC-I or MHC-II (Nielsen et al., (2004), Bioinformatics, 20 (9), 1388-1397), Bordner (2010), PLoS ONE 5(12): e14383) and how to evaluate binding of specific peptides (e.g. Bernardeau et al., (2011), J Immunol Methods, 371(1-2):97-105). Preferably, the T cell epitope is an MHC-I epitope. Preferably, the T cell epitope is an epitope derived from a cancer antigen; thus, preferably, the T cell epitope is an epitope essentially not or not occurring on normal cells of a subject, i.e. on non-cancer cells of said subject.

The term "T cell activating epitope", for which also the expression "activating epitope" may be used, is used herein in a broad sense to relate to any structure presented on the surface of a cell of a subject which can activate a T cell expressing an appropriate TCR. Preferably, the epitope is a polypeptide or fragment thereof, a polysaccharide, or a lipid. More preferably, the epitope is an epitope of a polypeptide presented by said cell of said subject in the context of an MHC molecule, preferably as specified herein above. As the skilled person understands, if a reactive T cell is identified by the method as specified herein in a sample, there preferably is a presumption that there are cells in said subject presenting a T cell activating epitope; since this identification not necessarily includes identifying the T cell activating epitope, the reactive T cell identified and/or its TCR may be used further for identifying the T cell activating epitope. Preferably, the T cell activating epitope is a cancer epitope. Thus, the reactive T cell may in particular be a cancer-reactive T cell or an autoimmune-reactive T cell.

The term "recognizing at least one epitope" is understood by the skilled person, and methods for determining whether a given T cell recognizes a given epitope are known in the art. Preferably, said recognizing is specific, i.e. preferably any other epitope is recognized less efficiently by a factor of at least 5, preferably at least 10, more preferably at least 20. Specificity of recognizing can be determined e.g. by determining activation of a T cell carrying a given TCR by a cognate epitope, e.g. when presented on a cell, and comparing said activation to an activation caused by a non-cognate epitope.

The terms "identifying a T cell reactive to target cells presenting a T cell activating epitope" and "identifying a reactive T cell", as used herein, are used in a broad sense including any and all means and methods of providing information on a reactive T cell allowing determination of at least the CDR3 sequences of its TCR, preferably of the CDR sequences of its TCR. In accordance, the reactive T cell does not have to, but may, be provided in physical form. Thus, identifying a reactive T cell may comprise identifying a dataset indicative of a T cell expressing at least one biomarker as specified elsewhere herein and, optionally, allocating at least the CDR3 sequences of the TCR of said reactive T cell. Preferably, said dataset is or was determined by single-cell determination of gene expression, preferably by single-cell RNA sequencing. Thus, step a) of the method of identifying a reactive T cell may comprise performing single-cell determination of gene expression of T cells in a sample, wherein expression of at least one of the biomarkers as specified is determined, thereby identifying a reactive T cell; optionally, at least the CDR3 sequences of the TCR of said T cell found to express said at least one biomarker are sequenced. Identifying a reactive T cell may, however, also comprise physically providing said reactive T cell. Thus, step a) of the method of identifying a reactive T cell may comprise determining expression of at least one of the biomarkers as specified on and/or in the T cell. Thus, expression of surface biomarkers may e.g. be determined by antibody staining, optionally followed by FACS-measurements and/or FACS sorting. Also preferably, single T cells are grown clonally and biomarker expression is determined in an aliquot of said clonally grown cells. Other methods of determining biomarker expression in a T cell, preferably a living T cell, are known in the art.

Determination of expression of a biomarker may be performed based on the amount of any biomarker gene product deemed appropriate by the skilled person. Thus, determination may comprise determining the amount of RNA, in particular mRNA, and/or polypeptide gene product. Expression may, however, also be determined by measuring expression of a surrogate biomarker, e.g. a reporter gene construct in which the reporter gene is expressed under the control of the promoter of the respective biomarker. Preferably, the determination of expression comprises determining the amount of mRNA and/or polypeptide gene product.

Identifying a reactive T cell comprises determining expression of at least one biomarker as specified elsewhere herein. Expression of a biomarker may be determined qualitatively, semi-quantitatively, or quantitatively, which terms are in principle known to the skilled person. Qualitative determination may be a binary assessment that the biomarker is expressed or not expressed by a T cell, e.g. by determining whether the biomarker is expressed above a detection level of an assay. Semiquantitative determination may comprise allocating an expression level to expression categories, such as low, medium, or high expression. The term quantitative determination is understood by the skilled person to include each and every determination providing information on the amount of a biomarker in a cell and all values derived from such an amount by at least one standard mathematical operation, including in particular calculation of a concentration, of a mean, a median, or an average, normalization, and similar calculations.

Preferably, identifying a reactive T cell comprises comparing biomarker expression determined in a T cell to a reference. The term "reference", as used herein, refers to expression of a biomarker in a reference cell, e.g. an amount of biomarker in a reference cell. Preferably, a reference is a threshold value (e.g., an amount or ratio of amounts) for a gene product. The reference may, however, also be a value derived from an amount by any mathematical calculation deemed appropriate by the skilled person, in particular normalization. In accordance with the aforementioned method, a reference is, preferably, a reference obtained from a sample of T cells known to be reactive T cells. In such a case, a value for the biomarker gene product found in a sample being essentially identical to said reference is indicative for a reactive T cell. Also preferably, the reference is from a sample of T cells known not be reactive. In such a case, a value for the biomarker gene product found in the T cell to be different with respect to the reference is indicative for the T cell being reactive. The same applies mutatis mutandis for a calculated reference, most preferably the average or median, for the relative or absolute value of the biomarker gene product(s) of a population of non-stimulated T cells. As the skilled per-son understands, only a small percentage of T cells of any given natural population of T cells will be reactive at a time. In accordance, the above description for a population of T cells known not to be activated may be applied mutatis mutandis to a natural population of T cells of which the activation status is unknown; thus the reference may be a natural sample of T cells of which reactivity status is unknown. In such a case, a value for the biomarker gene product found in the T cell to be different with respect to the reference is indicative for the T cell being reactive. How to calculate a suitable reference value, preferably, the average or median, is well known in the art. The population of non-stimulated T cells referred to before shall comprise a plurality of T cells, preferably at least 10, more preferably at least 100, still more preferably at least 1 ,000, most preferably at least 10,000, non-stimulated T cells. The value for a biomarker gene product of T cell of interest and the reference values are essentially identical if the corresponding values are essentially identical. Essentially identical means that the difference between two values is, preferably, not statistically significant. Preferably, the reference(s) are stored in a suitable data storage medium such as a database and are, thus, also available for future assessments.

Identifying a reactive T cell comprises determining expression of at least one biomarker selected from Table 1 herein below. The biomarkers of Table 1 are, as gene products, in principle known to the skilled person and their amino acid sequences and sequences of encoding polynucleotides are available from public databases. Each of the aforesaid biomarkers alone or any combination thereof is indicative of a reactive T cell. Preferably, however, at least 20, preferably at least 30, more preferably at least 42 of said biomarkers are determined. More preferably, at least 47 of said biomarkers are determined. Preferably, at most 1000, more preferably at most 250, still more preferably at most 100, even more preferably at most 50 biomarkers are determined.

Identifying a reactive T cell preferably comprises determining a probability parameter for at least one T cell; wherein the term "probability parameter" includes each and every parameter indicative of a probability of given T cell to be a reactive T cell. Thus, the method of identifying a reactive T cell described herein preferably provides a value of a probability parameter for at least one T cell. Thus, T cells preferably are not divided into a dichotomic distinction, i.e. activated or non-activated, but are allocated to a continuous scale of values of a probability parameter, e.g. from 0% to 100% probability of being activated, or any arbitrary scale deemed appropriate by the skilled person.

The term "cell" is understood by the skilled person to relate to any cell of the eukaryota, prokarya (bacteria), or archea domains. Preferably, the cell is a living cell, i.e. preferably capable of growth and/or proliferation under appropriate conditions. The term "host cell", as referred to herein, relates to any cell capable of expressing, and preferably presenting on its surface, a TCR polypeptide as specified herein, preferably encoded by a polynucleotide and/or vector. Preferably, the cell is a bacterial cell, more preferably a cell of a common laboratory bacterial strain known in the art, most preferably an Escherichia strain, in particular an E. coli strain. Also preferably, the host cell is a eukaryotic cell, preferably a yeast cell, e.g. a cell of a strain of baker's yeast, or is an animal cell. More preferably, the host cell is an insect cell or a mammalian cell, in particular a mouse or rat cell. Most preferably, the host cell is a human cell. Preferably, the host cell is a T cell, more preferably a CD8+ T cell or a CD4+ T cell, more preferably a CD8+ T cell. As the skilled person understands, a CD4 TCR is preferably expressed in a CD4+ T cell, and a CD8 TCR is preferably expressed in a CD8 T cell.

The term "target cell", as used herein, relates to a cell having the activity of presenting at least one T cell activating epitope on its surface. In accordance, the target cell expresses MHC-I and/or MHC-II, more preferably MHC-I. Thus, the target cell may be a cell of a body of a subject as specified elsewhere herein; i.e. preferably is a mammalian cell. The target cell may in particular be a cancer cell, preferably presenting at least one cancer antigen.

The term "cancer", as used herein, relates to a disease of an animal, including man, characterized by uncontrolled growth by a group of body cells ("cancer cells"). This uncontrolled growth may be accompanied by intrusion into and destruction of surrounding tissue (invasion) and possibly spread of cancer cells to other locations in the body (metastasis). Preferably, also included by the term cancer is a recurrence of cancer after treatment (relapse). Thus, preferably, the cancer is a solid cancer, a metastasis, or a relapse thereof. Cancer may be induced by an infectious agent, preferably a virus, more preferably an oncogenic virus, more preferably Epstein-Barr virus, a hepatitis virus, Human T-lymphotropic virus 1, a papillomavirus, or Human herpesvirus 8. Cancer may, however, also be induced by chemical compounds, e.g. a carcinogen, by physical factors, such as ionizing radiation, or endogenously, e.g. caused by spontaneous mutation. Preferably, the cancer comprises at least one cancer epitope.

Preferably, the cancer is selected from the list consisting of acute lymphoblastic leukemia, acute myeloid leukemia, adrenocortical carcinoma, aids-related lymphoma, anal cancer, appendix cancer, astrocytoma, atypical teratoid, basal cell carcinoma, bile duct cancer, bladder cancer, brain stem glioma, breast cancer, burkitt lymphoma, carcinoid tumor, cerebellar astrocytoma, cervical cancer, chordoma, chronic lymphocytic leukemia, chronic myelogenous leukemia, colon cancer, colorectal cancer, craniopharyngioma, endometrial cancer, ependymoblastoma, ependymoma, esophageal cancer, extracranial germ cell tumor, extragonadal germ cell tumor, extrahepatic bile duct cancer, fibrosarcoma, gallbladder cancer, gastric cancer, gastrointestinal stromal tumor, gestational trophoblastic tumor, hairy cell leukemia, head and neck cancer, hepatocellular cancer, hodgkin lymphoma, hypopharyngeal cancer, hypothalamic and visual pathway glioma, intraocular melanoma, kaposi sarcoma, laryngeal cancer, medulloblastoma, medulloepithelioma, melanoma, merkel cell carcinoma, mesothelioma, mouth cancer, multiple endocrine neoplasia syndrome, multiple myeloma, mycosis fungoides, nasal cavity and paranasal sinus cancer, nasopharyngeal cancer, neuroblastoma, non-hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, sézary syndrome, small cell lung cancer, small intestine cancer, soft tissue sarcoma, squamous cell carcinoma, squamous neck cancer, testicular cancer, throat cancer, thymic carcinoma, thymoma, thyroid cancer, urethral cancer, uterine sarcoma, vaginal cancer, vulvar cancer, waldenström macroglobulinemia, and wilms tumor. More preferably, the cancer is a solid cancer, a metastasis, or a relapse thereof. More preferably, said cancer is melanoma, brain cancer, preferably metastatic brain cancer, pancreatic cancer, lung cancer, rectal cancer, renal cancer, preferably metastatic renal cancer, or is gastrointestinal cancer.

The term "subject", as used herein, relates to an animal, preferably a vertebrate, more preferably a mammal, preferably to a livestock, like a cattle, a horse, a pig, a sheep, or a goat, to a companion animal, such as a cat or a dog, or to a laboratory animal, like a rat, mouse, or guinea pig. Preferably, the mammal is a primate, more preferably a monkey, most preferably a human. Preferably, the subject is suffering from cancer, in particular in case of the method of identifying a T cell reactive to cancer cells of a subject. It is, however, also envisaged that the subject is an apparently healthy subject, preferably at least 50 years, more preferably at least 60 years, more preferably at least 70 years, even more preferably at least 80 years of age.

The term "biomarker", as used herein, refers to a molecular species which serves as an indicator for a physiological state as referred to herein. Said molecular species can be a chemical compound which is detectable in a sample of a subject. Moreover, the biomarker may also be a molecular species which is derived from said chemical compound. In such a case, the actual metabolite will be chemically modified in the sample or during the determination process and, as a result of said modification, a chemically different molecular species will be the determined molecular species. E.g., in case the biomarker is a polypeptide or protein, the analyte may be a derivative of the polypeptide or protein, may be a fragment of the polypeptide or protein, or may be a complex of the polypeptide, e.g. an immunocomplex of the polypeptide or a protein comprising more than one polypeptide. Also, the biomarker may be a surrogate marker, i.e. a marker different from the actual biomarker, but nonetheless indicative of the presence and optionally the amount of the biomarker. E.g. the amount of an encoding mRNA may be used as a surrogate marker in case the biomarker is a polypeptide produced by a cell. Further, in case the biomarker has an activity, e.g. a catalytic activity and/or an activating activity, e.g. on immune cells or on target cells, the biomarker may also be determined via said activity, e.g. in an enzymatic assay or an activation assay. Moreover, as is understood by the skilled person, a biomarker according to the present invention need not necessarily correspond to one molecular species. Rather, e.g. in case the biomarker is a polypeptide, the biomarker may comprise polypeptide variant molecular species, e.g. translated from splice variants, glycosylation variants, peptidase processing variants, and the like, wherein, preferably, the variants share at least one determinable feature, e.g. an epitope or an activity.

The biomarkers of the present invention are summarized in Table 1 below. The biomarkers of Table 1 are, in principle known to the skilled person and can be accessed by the indicated accession numbers. Unless indicated otherwise, all database accession numbers in this description refer to the respective entry current at the filing date of this description. It is understood by the skilled person that the biomarkers are referenced as biomarkers, not as specific polynucleotides or polypeptides. Accordingly, the biomarkers, in particular polypeptides having the specific sequences deposited under the database accession numbers, are preferably to be understood as exemplary sequences representing a biomarker. Encompassed as gene products as specified herein are also polypeptide variants, encoding polynucleotides, in particular mRNA, and any other gene products deemed appropriate by the skilled person in the context of the present description.

Preferably, step (a) comprises determining at least one biomarker selected from the group consisting of MAGEH1, HMGB2, MIR4435-2HG, TMSB10, MGAT4A, C12orf57, GIMAP7, HSPE1, IL6ST, STMN1, IL32, TPT1, LYAR, MIF, PALM2-AKAP2, ISCU, CCR8, UGP2, EEF1B2, SETD7, CRIP1, RGS1, MESD, LINC02099, IFT52, AKIRIN2, RIOK3, ANKRD12, CALR, DDX5, CNST, GNPDA1, SPOCK2, MALAT1, TOX, RGL4, B2M, IFITM2, ABI3, PTPN11, BTG1, ITSN2, RAD21, FYB1, DDX21, HSPD1, JAK1, ADAM19, TM2D3, CASP8, CARS1, PTPN7, HAPLN3, CSTB, TLE5, ACTG2, IL2RA, CD99, CARHSP1, TMSB4X, CCR4, and PCM1.

As the skilled person understands, identifying a reactive T cell may comprise determining expression of a plurality of biomarkers, e.g. least 20, preferably at least 30, more preferably at least 38, most preferably at least 48, of said biomarkers are determined. In such case, expression of at least one biomarker selected from the group consisting of CXCL13, ANXA1, FOXP3, AREG, AC243829.4, IL6ST, CLIC3, IFNG, CTSC, CRIP1, CTLA4, RBPJ, IL7R, ENTPD1, KIR2DL4, ABI3, NKG7, PRF1, LMNA, LINC02446, PKM, RSRP1, IKZF3, SPOCK2, TOX, MIR4435-2HG, CD63, LYAR, KLRD1, PSMB9, CARS, LTB, KLF6, APOBEC3G, CARHSP1, RHOH, VAMP5, ACP5, NR4A3, CST7, JUNB, CHST12, RABAC1, TSEN54, GALNT2, and LINC01871 may be determined in addition to the at least one biomarker as specified herein above.

Also preferably, at least ten biomarkers are determined selected from the group consisting of MAGEH1, HMGB2, MIR4435-2HG, TMSB10, MGAT4A, C12orf57, GIMAP7, HSPE1, IL6ST, STMN1, IL32, TPT1, LYAR, MIF, PALM2-AKAP2, ISCU, CCR8, UGP2, EEF1B2, SETD7, CRIP1, RGS1, MESD, LINC02099, IFT52, AKIRIN2, RIOK3, ANKRD12, CALR, DDX5, CNST, GNPDA1, SPOCK2, MALAT1, TOX, RGL4, B2M, IFITM2, ABI3, PTPN11, BTG1, ITSN2, RAD21, FYB1, DDX21, HSPD1, JAK1, ADAM19, TM2D3, CASP8, CARS1, PTPN7, HAPLN3, CSTB, TLE5, ACTG2, IL2RA, CD99, CARHSP1, TMSB4X, CCR4, and PCM1.

More preferably, at least ten, preferably at least 20, more preferably at least 30, even more preferably at least 38, still more preferably at least 48, most preferably all, biomarkers of Table 1 are determined; in such case, also more preferably, at most 1000, still more preferably at most 250, even more more preferably at most 100, biomarkers are determined.

Preferably, the method does not comprise determining ABI3, AC243829.4, ACP5, AKAP13, ANXA1, APOBEC3G, AREG, CARHSP1, CCL3, CCNH, CD27, CD63, CD7, CHST12, CLIC3, CRIP1, CST7, CTLA4, CTSC, CXCL13, FOXP3, FTH1, GALNT2, GZMA, HLA-DRA, HMOX1, IFNG, IKZF3, IL6ST, IL7R, KIR2DL4, KLF6, LINC02446, LTB, LYAR, MIR4435-2HG, NKG7, PKM, PRF1, PSMB9, RABAC1, RBPJ, RGS1, RHOH, RSRP1, SPOCK2, and/or TOX.

The term "sample" refers to a sample of biological material comprising cells and known or suspected to comprise T cells. The sample may, in principle, be of any type deemed appropriate by the skilled person. Preferably, the sample is a sample from a subject. The sample may be a sample of a bodily fluid, such as blood, urine, saliva, and the like, or a sample derived from a bodily fluid, such as e.g. plasma or serum. Preferably, the sample is a sample of separated cells or a sample from a tissue or an organ, preferably from a tumor. As is known to the skilled person, tissue or organ samples may be obtained from any tissue or organ by, e.g., biopsy, surgery, or any other method deemed appropriate by the skilled person. Separated cells may be obtained from body fluid samples, such as lymph, blood, plasma, serum, liquor and other, or from the tissues or organs by separating techniques such as centrifugation or cell sorting. Preferably, the sample is a tissue or body fluid sample which comprises cells. Also preferably, the sample is a sample of a brain metastasis of a non-brain primary tumor.

The method for determining a reactive T cell comprises step (a) determining expression of at least one biomarker selected from the group consisting of MAGEH1, HMGB2, TMSB10, MGAT4A, C12orf57, GIMAP7, HSPE1, STMN1, IL32, TPT1, MIF, PALM2-AKAP2, ISCU, CCR8, UGP2, EEF1B2, SETD7, MESD, LINC02099, IFT52, AKIRIN2, RIOK3, ANKRD12, CALR, DDX5, CNST, GNPDA1, MALAT1, RGL4, B2M, IFITM2, PTPN11, BTG1, ITSN2, RAD21, FYB1, DDX21, HSPD1, JAK1, ADAM19, TM2D3, CASP8, CARS1, PTPN7, HAPLN3, CSTB, TLE5, ACTG2, IL2RA, CD99, TMSB4X, CCR4, and PCM1 in T cells from a sample of said subject.

The method for determining a reactive T cell may alternatively comprise step (a) determining expression of at least 10 biomarkers selected from the group consisting of CXCL13, AREG, MAGEH1, FOXP3, ANXA1, HMGB2, MIR4435-2HG, TMSB10, MGAT4A, C12orf57, GIMAP7, HSPE1, IL6ST, STMN1, AC243829.4, IL32, LINC02446, IFNG, NKG7, CTSC, TPT1, LYAR, MIF, PALM2-AKAP2, ISCU, CCR8, UGP2, EEF1B2, SETD7, LTB, CRIP1, APOBEC3G, RBPJ, RGS1, MESD, LINC02099, IKZF3, IFT52, AKIRIN2, RIOK3, CLIC3, ANKRD12, CALR, DDX5, CNST, GNPDA1, PKM, SPOCK2, RSRP1, IL7R, KIR2DL4, MALAT1, CTLA4, TOX, RGL4, KLF6, B2M, IFITM2, ABI3, CST7, PTPN11, BTG1, CCNH, ITSN2, RAD21, CCL3, HMOX1, FYB1, DDX21, GALNT2, HSPD1, GZMA, JAK1, CHST12, ADAM19, PRF1, CD63, TM2D3, CASP8, CARS1, ACP5, PTPN7, RHOH, HAPLN3, CSTB, TLE5, ACTG2, CD7, IL2RA, CD99, CARHSP1, TMSB4X, PSMB9, CCR4, AKAP13, RABAC1, HLA-DRA, FTH1, CD27, and PCM 11, in T cells from a sample of said subject.

Other options for biomarkers and their combinations which may be determined have been described herein above.

The method of identifying a reactive T cell comprises step (b) identifying a reactive T cell based on the determination of step (a). Appropriate methods for identifying have been described herein above. Preferably, the method further comprises step (b1) comparing the result of the determining in step (a) to a reference as specified herein above.

Advantageously, it was found in the work underlying the present invention that the markers described herein are particularly suitable for identifying reactive T cells and, thus, TCRs of reactive Tcells.

The definitions made above apply mutatis mutandis to the following. Additional definitions and explanations made further below also apply for all embodiments described in this specification mutatis mutandis.

The present invention also relates to a method of identifying a TCR binding to an activating epitope presented on a cell, preferably a cancer cell, of a subject, said method comprising
(A) identifying a reactive T cell according to the method described herein,
(B) providing the amino acid sequences of at least the complementarity determining regions (CDRs) 3, preferably of CDRs 1 to 3, of the TCR of the reactive T cell identified in step (A); and, hereby,
(C) identifying a TCR binding to an activating epitope presented on a cell.

The term "identifying a TCR" is used herein in a broad sense including any and all means and methods of providing information on a TCR allowing determination of at least its CDR3 sequences, preferably its CDR sequences. In accordance, the TCR does not have to, but may, be provided in physical form. Thus, identifying a TCR may comprise providing at least the CDR3 sequences of the TCR or of a polynucleotide encoding at least said CDRs. Preferably, said sequences are or were determined by single-cell determination of gene expression, preferably by single-cell RNA sequencing, preferably as specified herein above. Identifying a TCR may, however, also comprise physically providing said TCR, e.g. by providing a host cell, preferably a T cell, expressing said TCR, or by providing at least one polynucleotide encoding at least the CDRs of the TCR polypeptides identified. As will be understood, in case the TCR is provided in the context of a self-replicating entity such as a host cell, it may not be necessary to provide the amino acid sequence of at least the CDRs of the TCR and/or the nucleic acid sequence of a polynucleotide encoding the same.

The term "providing a sequence", such as an amino acid sequence and/or a nucleic acid sequence, is used herein in a broad sense including any and all means and methods of providing information on said sequence or making said sequence information available. Thus, the sequence may be provided as a sequence information, preferably tangibly embedded on a data carrier. The sequence may, however, also be provided in the form of a molecule comprising said sequence, preferably as a TCR comprising TCR alpha and beta chains comprising said sequences, more preferably as a host cell comprising said TCR. As the skilled person under-stands, if the aforesaid host cell is provided, the sequence information can be provided by standard methods known to the skilled person, e.g. nucleic acid sequencing of the TCR ex-pressed by said host cell or of parts thereof.

The method of identifying a TCR comprises step (A) identifying a reactive T cell according to the method described herein. The method of identifying a TCR further comprises step (B) providing the amino acid sequences of at least the complementarity determining regions (CDRs) 3 of the TCR of the reactive T cell identified in step (A). Suitable methods for providing an amino acid sequence of at least the CDR3s of a TCR have been described herein above.

Preferably, the method of identifying a TCR binding to an activating antigen further comprises step (B1) expressing a TCR comprising at least the CDR3s determined in step (B) in a host cell, preferably a T cell. More preferably, said method comprises further step (B1) expressing a TCR comprising at least the CDR3s determined in step (B) in a host cell, preferably a T cell, i.e. preferably comprises expressing a TCR comprising at least the CDR3s determined in step (B) and at least one accessory TCR polypeptide in a host cell.

The term "TCR comprising at least the CDR3s", as used herein, relates to a TCR in which at least the CDR3s are those as determined in step (B), while the residual sequences of the TCR polypeptides may be sequences of one or more different alpha and beta or gamma and delta chains, e.g. heterologous sequences. More preferably, the variable regions of the TCR molecules are provided in step (B) and are expressed as parts of the TCR polypeptides in step (B1). It is, however, also envisaged that sequences of further fragments of the TCR polypeptides or the complete TCR polypeptides are provided in step (B), and are optionally expressed in step (B1). As the skilled person understands, it is also possible to provide longer sequences in step (B) than are expressed in step (B1); e.g., preferably, the amino acid sequence of the variable regions of the TCR polypeptides may be provided in step (B), while only the CDRs thereof are expressed, in the context e.g. of heterologous TCR polypeptides, in step (B1); or, the amino acid sequences of the variable regions of the TCR polypeptides may be provided in step (B), and the amino acid sequence of the antigen binding region, including the CDRs, may be expressed, in the context e.g. of heterologous TCR polypeptides, in step (B1). If not otherwise indicated, the TCR polypeptides preferably are expressed as complete molecules, i.e. each comprising a transmembrane region, a constant region, a joining region, and a variable region.

Preferably, the method of identifying a TCR binding to a cell presenting a T cell activating antigen comprises further step (B2) determining binding of the TCR expressed in step (B1) to a cell presenting a T cell activating antigen, preferably a cancer antigen, complexed in a major histocompatibility complex (MHC), preferably MHC class I, molecule. Methods of determining binding of a TCR, preferably comprised in a TCR, to a T cell activating antigen complexed in a major histocompatibility complex (MHC) molecule are known in the art and include, preferably, determining binding of a T cell activating antigen complexed an MHC molecule carrying a detectable label to the TCR which may e.g. be expressed on the surface of a host cell. A well-known example of such a method is a tetramer assay, preferably using a soluble tetrameric MHC molecule complexed with a T cell activating antigen.

Preferably, the method for identifying a TCR binding to a T cell activating antigen comprises further step (B3) determining recognition of cells presenting said T cell activating antigen by the TCR expressed in step (B1). Assays for determining such recognition are known in the art and include in particular binding assays, activation assays, and lysis assays. In all of these assays, preferably cells presenting a T cell activating antigen are co-incubated with host cells such as T cells expressing a TCR comprising at least the CDR3s as specified. In a binding assay, it is determined whether the target cell, e.g. cancer cells, and the aforesaid host cells bind to each other, preferably to form an immunological synapse including at least an MHC molecule of the target cell and the TCR of the T cell. In an activation assay, the host cell, preferably the T cell, expressing a TCR comprising at least the CDR3s as specified, is tested after said co-incubation for biomarkers of immunological activation, e.g. interferon-gamma production. In a lysis assay, it is determined whether the host cells, preferably the T cells, expressing a TCR comprising at least the CDR3s as specified, lysed at least a fraction of the cells presenting the T cell activating antigen, i.e. target cells, during said co-incubation.

Preferably, the method of identifying a TCR binding to an activating antigen comprises further step (B4) producing a soluble TCR comprising at least the CDR3s determined in step (B) and determining binding of said soluble TCR to a cancer cell and/or to a cancer antigen complexed in a major histocompatibility complex (MHC), preferably MHC class I, molecule. Soluble TCRs have been described herein above. Preferably, soluble TCRs carrying a detectable label are used in step (B4); thus, binding of a such labeled soluble TCR may e.g. be detected by fluorescence-activated cell sorting equipment.

Preferably, in the method of identifying a TCR, expression of said at least one biomarker is determined by single-cell determination of gene expression, preferably of at least 100 T cells, more preferably at least 1000 T cells. In such case, the amino acid sequences of at least the complementarity determining regions (CDRs) of the TCR of the reactive T cell of step (B) may be provided as part of the single-cell determination of gene expression, i.e. the mRNAs encoding said CDRs may be sequenced as part of said single-cell determination of gene expression. Preferably, corresponding sequences are pre-amplified before single-cell determination of gene expression. The mRNAs encoding said CDRs may, however, also be determined in separate sequencing steps, preferably by using appropriate barcoding methods. Also in the aforesaid case, the method may comprise further step (B*1) clustering the T cells based on said gene expression data including the amino acid sequences of said at least CDR sequences and further step (B*2) selecting the TCR or TCRs being clustered at increased relative frequency in clusters expressing said at least one biomarker compared to clusters not expressing said at least one biomarker.

The method of identifying a TCR further comprises the identification step, i.e. step (C) identifying a TCR binding to an activating epitope presented on a cell, preferably as described herein elsewhere.

The present invention also relates to a method of providing a T cell recognizing a cell presenting a T cell activating epitope, preferably a cancer cell, said method comprising
(i) identifying a TCR binding to a cell presenting a T cell activating epitope according to the method according to the method of identifying a TCR described herein,
(ii) expressing a TCR comprising at least the complementarity determining regions (CDRs) 3 of the TCR of step (i) in a T cell, and, thereby,
(iii) providing a T cell recognizing a cell presenting a T cell activating epitope, preferably a cancer cell.

The method of providing a T cell may comprise further steps in addition to those related to herein above. For example, further steps may relate, e.g., to cloning at least polynucleotides encoding the CDR3s of the TCR of step (i) into a TCR alpha, beta, gamma, or delta chain backbone, or cloning polynucleotides encoding the variable regions of the TCR polypeptides of step (i) into TCR alpha and beta or a TCR gamma and delta, chain backbones, preferably on at least one expression vector; or cloning polynucleotides encoding TCR polypeptides into one or more expression vectors. As will be understood by the skilled person, CDRs and/or a variable region of a TCR alpha chain will preferably be cloned into a TCR alpha chain backbone; and CDRs and/or a variable region of a TCR beta chain will preferably be cloned into a TCR beta chain backbone. The aforesaid applies mutatis mutandis to gamma and delta chains. The method may also comprise the further step of expanding, preferably clonally expanding, the T cell recognizing a cancer cell to provide a preparation of cells T cell recognizing cancer cells. It will thus be appreciated that the T cell recognizing cancer cells may be the T cell identified in step (i) or a clonal derivative (i.e. a daughter cell) thereof.

Preferably, the method of providing a T cell further comprises a step of testing reactivity of the T cell of step (ii) to cells presenting an activating agent, e.g. a cancer cells. The term "testing reactivity of a T cell", as used herein, includes each and every method deemed suitable by the skilled person to determine whether the T cell as specified is reactive. Preferred methods for testing reactivity have been described herein above, e.g. determining binding, activation of T cells, and/or lysis of cells presenting an activating antigen, e.g. cancer cells.

The present invention further relates to a reactive T cell identified by the method of identifying a T cell reactive to cells presenting a T cell activating epitope, preferably cancer cells, as specified herein above and/or obtained or obtainable by the method of providing a T cell recognizing a cells presenting a T cell activating antigen as specified herein above; and to the aforesaid reactive T cell for use in medicine, in particular for use in treating and/or preventing cancer or auto-immune disease in a subject.

The terms "treating" and "treatment" refer to an amelioration of the diseases or disorders referred to herein or the symptoms accompanied therewith to a significant extent. Said treating as used herein also includes an entire restoration of health with respect to the diseases or disorders referred to herein. It is to be understood that treating, as the term is used herein, may not be effective in all subjects to be treated. However, the term shall require that, preferably, a statistically significant portion of subjects suffering from a disease or disorder referred to herein can be successfully treated. Preferably, treating cancer is reducing cancer, e.g. tumor, burden in a subject. As will be understood by the skilled person, effectiveness of treatment of e.g. cancer is dependent on a variety of factors including, e.g. cancer stage and cancer type.

The term "preventing" refers to retaining health with respect to the diseases or disorders referred to herein for a certain period of time in a subject. It will be understood that the said period of time may be dependent on the amount of the drug compound which has been administered and individual factors of the subject discussed elsewhere in this specification. It is to be understood that prevention may not be effective in all subjects treated with the compound according to the present invention. However, the term requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification.

The present invention further relates to a polynucleotide encoding at least one TCR binding to an activating epitope, wherein said TCR was provided or identified according to the method described herein.

The present invention also relates to a device or kit comprising the reactive T cell described herein.

The term "device", as used herein relates to a system of means comprising at least the means described, preferably operatively linked to each other and/or further means such as to allow administration of the T cell. Preferred means for administering a T cell are well known in the art and are described herein above. How to link the means in an operating manner will depend on the type of means included into the device and on the kind of administration envisaged. Preferably, the means are comprised by a single device in such a case. Said device may accordingly include a delivery unit for the administration of the T cell and, optionally, a storage unit for storing said T cell until administration. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician. Preferably, the device is a syringe, more preferably with a needle, comprising the T cell. More preferably, the device is an intravenous infusion (IV) equipment comprising the T cell. Also preferably, the device further comprises a needle for topical application of the T cell, e.g. to tumor.

The term "kit", as used herein, refers to a collection of the aforementioned compounds, means or reagents which may or may not be packaged together. The components of the kit may be comprised by separate vials (i.e. as a kit of separate parts) or provided in a single vial, e.g. as a composition as specified herein above. The components of the kit are preferably comprised in a housing, wherein the housing preferably allows translocation of the compounds of the kit, in particular common translocation; thus, the housing may in particular be a transportable container comprising all specified components. Preferably, the kit is adapted for use in a method of the present invention, more preferably is adapted to comprise all reagents required to perform said method or methods.

The present invention also relates to a method for treating and/or preventing cancer in a subject, said method comprising
(a) administering a reactive T cell described herein to said subject; and
(b) thereby treating and/or preventing cancer in said subject.

The present invention also relates to a method for treating and/or preventing cancer in a subject, said method comprising
(A) providing or having provided a T cell recognizing a cell presenting a T cell activating epitope described herein;
(B) administering the reactive T cell of step (A) to said subject; and
(C) thereby treating and/or preventing cancer in said subject.

The present invention also relates to a use of a method described herein and/or a reactive T cell described herein for providing a medicament, wherein said medicament preferably is for use in treating cancer.

The terms "medicament" and "pharmaceutical composition" are used essentially interchangeably herein and are, in principle, known to the skilled person. As referred to herein, the terms relate to any composition of matter comprising the specified active agent(s), in particular T cells, as pharmaceutically active compound(s) and one or more excipient. It will be appreciated that the form and character of the pharmaceutical acceptable excipient, e.g. carrier or diluent, is dictated by the amount of active ingredient with which it is to be combined, the route of administration, and other well-known variables. The excipient(s) must be acceptable in the sense of being compatible with the other ingredients of the formulation and being not deleterious to the recipient thereof. Thus, the excipient employed preferably is a liquid, preferably an aqueous solution. Exemplary of liquid carriers are phosphate buffered saline solution, physiological saline, Ringer's solutions, dextrose solution, Hank's solution,, and the like. Said suitable carriers comprise those mentioned above and others well known in the art, see, e.g., Remington's Pharmaceutical Sciences, Mack Publishing Company, Easton, Pennsylvania. The excipient(s) is/are selected so as not to compromise the biological activity of the combination and of the T cells comprised therein.

The medicament is, preferably, administered locally, topically or systemically, preferably is administered systemically. Suitable routes of administration of the pharmaceutical composition are inhalation, oral, intravenous, intramuscular, subcutaneous, rectal, transdermal, as well as parenteral administration. Preferably, the inhibitory polypeptide or the pharmaceutical composition are administered systemically or topically, e.g. intratumorally or intracranially.

A therapeutically effective dose refers to an amount of T cells to be used in a medicament which prevents, ameliorates or cures the symptoms accompanying a disease or condition referred to in this specification. As is well known in the medical arts, dosages for any one patient depend upon many factors, including the patient's size, age, the particular formulation of the medicament to be administered, sex, time and route of administration, general health, and other drugs being administered concurrently. The medicament referred to herein is, preferably, administered at least once, e.g. as a bolus. However, the medicament may be administered more than one time and, preferably, at least twice, e.g. permanently or periodically after defined time windows. Progress can be monitored by periodic assessment. Dosage recommendations may be indicated in the prescriber or user instructions in order to anticipate dose adjustments depending on the considered recipient.

The medicament may comprise further active agents in addition to the aforementioned active agent(s). Preferably, the pharmaceutically active compound is to be applied together with at least one further drug and, thus, may be formulated together with this at least one further drug as a medicament. More preferably, in case of cancer treatment, said at least one further active agent is a further immunotherapeutic agent, such as an immune checkpoint modulator. Also, it is to be understood that the formulation of a pharmaceutical composition preferably takes place under GMP standardized conditions or the like in order to ensure quality, pharmaceutical safety, and effectiveness of the medicament.

The present invention further relates to a computer-implemented method of training at least one trainable model for identifying a T cell reactive to target cells of a subject presenting a T cell activating epitope (reactive T cell), the method comprising:
(A) providing the trainable model;
(B) retrieving allocated training data, the allocated training data comprising gene expression data of T cells allocated to reactivities of the T cell receptors (TCRs) of said T cells to said target cells, wherein said gene expression data and said reactivities of the TCRs were obtained by the method described herein above; and
(C) training the trainable model on the allocated training data.

The aforesaid method preferably is an in silico method. Also, the method may comprise any additional steps deemed appropriate by the skilled person, e.g. further step (D) thereby providing a trained model for identifying a reactive T cell.

The term "training", as used herein, includes each and every process of establishing parameters of at least one machine learning and/or deep learning model, preferably of an algorithm of said machine learning and/or deep learning model, preferably on at least one training data set, which may also be referred to as set of training data. The training preferably comprises at least one optimization or tuning process, wherein a suitable, preferably best, parameter combination, e.g. according to at least one optimization procedure, is determined.

The training method preferably further comprises at least one hyperparameter tuning step for tuning hyperparameters of the trainable model, the term "hyperparameter tuning step" preferably relating to the process of adjusting, preferably optimizing, one or more hyperparameters of the trainable model. Thus, trainable models, e.g. trainable models from standard software libraries such as xgboost or scikit-learn, typically require one or more hyperparameters to be predetermined before starting the training process. Preferably, the hyperparameter tuning comprises Bayesian optimization using scikit-optimize with 10 stratified k-fold cross validation. The hyperparameter tuning step may comprise the pre-adjusting of these hyperparameters, e.g. in order to provide a suitable convergence for the training process. As an example, the allocated training data or at least a part thereof may be used for an initial test for training the trainable model, trying different sets of hyperparameters, wherein the performance of the training process is evaluated on the basis of measurable training performance values such as the speed of convergence, and, wherein, the set of hyperparameters providing a superior performance may finally be chosen for the trainable model. The trainable model may then be trained with this specific set of hyperparameters. The hyperparameter tuning step preferably is performed at least once, preferably before performing step (C). It is, however, also possible to implement a plurality of tuning steps, intermitting with one or more partial training steps, e.g. in order to improve or even optimize the tuning of the hyperparameters during the process.

The term "trainable model", as used herein, relates to any at least one mathematical model configured for transforming one or more input values into one or more output values by using one or more parameters which may be adjusted in order to enable the model to be trained. The trainable model preferably is a trainable mathematical model which is trainable on at least one training data set using one or more of machine learning, deep learning, neural networks, or other form of artificial intelligence. The trainable model preferably can be further trained, optimized or updated based on additional training data. Preferably, the trainable model is trained on a training dataset. The trainable model may be trained by using machine learning. The trainable model may be at least partially data-driven by being trained on data from historical training data.

The trainable model preferably comprises at least one trainable model selected from the group consisting of: a trainable XGBoost model, a trainable Adaboost model, a trainable Decision Tree model, a trainable Catboost model, a trainable Gaussian Naive Bayes model, a trainable K-nearest neighbour model, a trainable Logistic regression model, a trainable Radial Basis Function (RBF) Support Vector Machine (SVM) model, a trainable Random Forest model, and a trainable linear SVM model. Preferably, the trainable model is trainable XGBoost model.

The term "retrieving", as used herein, relates to any process of obtaining data from a data source. The data source may vary in accordance with the specific application. Thus, in the context of the description herein, retrieving the allocated training data may take place by at least one of the following: downloading the allocated training data from at least one data source, such as from at least one data storage device, from a web- or cloud-based data storage device; obtaining the allocated training data via at least one computer network, such as the Internet; obtaining the allocated training data via at least one wire-based and/or wireless interface. Retrieving of training data may fully or partially take place automatically, such as by automatic download of the allocated training data from public databases, and/or may fully or partially take place manually. Semi-automatic retrieving processes are also possible. Thus, as an example, data from at least one public database may be downloaded, e.g. automatically, and the data may be processed in order to generate the labelled training patient data, e.g. by manual allocation of the training data downloaded. Additionally or alternatively, retrieving the allocated training data may also comprise the actual measurement of the data, e.g., by evaluating one or more samples, such as by determining gene expression data by appropriate measurements.

The term "gene expression data", as used herein, relates to all data pertaining to expressed genes in a biological entity, such as a cell or tissue, preferably in a T cell. Typically, gene expression data are indicative of the abundance of the at least one gene product of at least one expressed genes, wherein said gene product may in particular be a polynucleotide, such as an mRNA, or a polypeptide, e.g. of a biomarker. As is known to the skilled person, a particular gene may give rise to more than one polynucleotide gene product, e.g. by alternative splicing, mRNA editing, by being alleles present in the cell, and the like; also, a particular gene may give rise to more than one polypeptide gene product, e.g. via alternative splicing, protein editing, glycosylation, proteolytic processing, and the like. Thus, preferably all gene products originating from one gene are assigned to said gene. It may, however, also be envisaged to assign different gene products to subclasses of one gene, in particular in case said different gene products have different functions in the cell. Also, it may be envisaged to determine only a part of the possible gene products of a gene, e.g. only one gene product, such as the most abundant gene product. Methods for determining abundance of one or more gene products are known in the art and are described elsewhere herein. Preferably, said method for determining abundance of one or more gene products is a method allowing for determining a plurality of gene products in one assay. Thus, in case the genes from which the products shall be determined have not been pre-determined, omics methods, such as transcriptomics or proteomics methods may be used; e.g. abundance of RNAs may be determined by one of the next generation and/or deep transcript sequencing methods known in the art. Based on the nucleic acid sequences of the transcripts or polypeptides of the expressed genes, the encoding genes may be identified, e.g., by comparing the nucleic acid sequences to those nucleic acid sequences of known genes contained in databases. The expressed gene can be identified based on a sequence match or essentially match in a sequence comparison. The name or designation of the identified expressed gene preferably is allocated to the gene expression data as referred to herein. The gene expression data may, in principle, be qualitative data indicating whether a gene is expressed or not, e.g. by determining whether a gene product is detectable above the detection limit of the detection method used. More preferably, the gene expression data are quantitative data comprising information on the abundance of the gene product(s) of the expressed gene, i.e. information as to the amount of gene product determined. In case the genes from which the products shall be determined have been pre-determined, the aforesaid methods may be used as well; however, also methods using specific probes, such as RNA array or protein array methods may be used. Description relating to determining expression of a biomarker herein above applies to determining expression of a gene product mutatis mutandis.

The gene expression data are allocated the reactivities of TCRs of T cells to target cells. As used herein, the term "reactivity of a TCR" relates to a propensity of a TCR to bind a predetermined epitope presented via an MHC molecule. Methods for determining said propensity are known to the skilled person and include, on an exemplary basis, determining binding of a TCR to a cell, e.g. by using a labeled soluble TCR in a FACS assay; determining activation of a T cell carrying said TCR by a target cell presenting a pre-determined epitope, and/or determining lysis of a target cell presenting a pre-determined epitope by a T cell carrying said TCR. In accordance with the above, the T cells used for determining gene expression data may be enriched for T cells carrying a reactive TCR; said enrichment may be natural enrichment, e.g. by using T cells from a sample known or expected to be enriched for reactive T cells, such as a tumor sample, or by using a sample from a vaccinated subject; said enrichment may, however, also be in vitro enrichment, e.g. by MHC-tetramer staining followed by FACS sorting, by MHC tetramer panning, and/or by cultivation of T cells including, preferably repeated, stimulation with one or more target epitope(s).

The term "allocated", as referred to herein, relates to the property of training data having, besides the gene expression data, additional information permitting a classification of the data. Thus, as an example, the allocated training data may contain, preferably for each patient or each data set of a patient, besides the gene expression data, an indication of the known reactivity and/or activation state of the T cell the gene expression data were obtained from, and/or the reactivity of the corresponding TCR. Thus, as an example, for each T cell comprising a given TCR, besides the gene expression data, further information may be comprised, e.g. indicating the reactivity of the TCR, and/or the reactivity and/or activation state of the T cell comprising said TCR.

Consequently, the allocated training data may be obtained by using data from a plurality of T cells. As an example, data from at least 10 TCRs and/or T cells, preferably data from at least 50 TCRs and/or T cells or more, may be used for generating the allocated training data. Preferably, in case the allocated training data comprise allocated data from TIL, the number of T cells from apparently healthy subjects represented is at least 5fold, preferably at least 10fold, more preferably at least 25 fold, even more preferably at least 50fold, most preferably at least 75 fold, the number of TILs represented. For each T cell, on the one hand, gene expression data may be generated. Further, for each T cell, information on TCR reactivity of the respective T cell may be obtained. As outlined above, also indicators other than gene expression data may be used for obtaining the TCR reactivity of the respective T cell, such as cell surface protein expression levels such as those measured using CITEseq to include common markers of immune cell phenotype such as CD4, CD8, commonly known activation markers, such as CD107a, NFAT, and/or CD69, as well as common markers of immune cell exhaustion such as PD1, CTLA4, and optionally secretion of at least one chemokine, migration behaviour, and the like. Then, for each T cell, the gene expression data are allocated at least with the respective information on the TCR reactivity of the respective T cell, in order to obtain an allocated training data set for the respective T cell. The aggregate of the allocated training data set of the TCRs and/or T cells may then form or form part of the allocated training data.

The term "trained model", as used herein, relates to a trainable model which has gone through at least one training process as defined above, by applying, at least once, a set of training data to the trainable model. Preferably, one or more parameters of the trainable model have been adapted during training. Preferably, the trained model is a trainable model which was trained on at least one training dataset. The trained model preferably is or comprises a classifier, configured for classifying an object, on the basis of one or more input variables, describing the object. The trained model preferably comprises at least one trained model selected from the group consisting of: a trained XGBoost model, a trained Adaboost model, a trained Decision Tree model, a trained Catboost model, a trained Gaussian Naive Bayes model, a trained K-nearest neighbour model, a trained Logistic regression model, a trained Radial Basis Function (RBF) Support Vector Machine (SVM) model, a trained Random Forest model, and a trained linear SVM model. Preferably, the trained model is a trained XGBoost model.

The trainable model and/or the trained model preferably may be o rmay be derived from a model taken from a software library, providing a plurality of trainable models. As an example, the Scikit-learn open-source software library may be mentioned for trainable models and/or trained models being programmed in the Python programming language. Additionally or alternatively, also, as an example, reference may be made to the eXtreme Gradient Boosting (XGBoost) open-source software library, preferably providing gradient boosting trainable models, being programmed in one or more of the programming languages C++, Java, Python, R, Julia, Perl and Scala. Other libraries, however, are also feasible, as well as customized trainable models not retrieved from software libraries.

The term "to classify" is known to the skilled person and preferably relates to any process of assigning an object to one or more predetermined or determinable classes. The classifying preferably comprises a prediction or assignment providing an indication to which class out of a plurality of classes an object belongs, e.g. in accordance with one or more predetermined properties of the object.

As outlined above, the trained model is configured for classifying a TCR and, optionally, a T cell carrying said TCR, into a reacticity state. Thus, as discussed above, the trained model preferably assigns the TCR and optionally the T cell to one reactivity state selected from a predetermined group of at least two (e.g. reactive vs. non-reactive), preferably at least three (e.g. low, intermediate, and strong reactivity), more preferably at least four (e.g. quartiles of reactivity), reactivity states. As indicated elsewhere herein, classification may also be in a continuous parameter, such as a probability of reactivity.

As also outlined above, the trainable model preferably may comprise or may consist of a trainable XGBoost model, i.e. a trainable model implementing a gradient boosting method. As also outlined in further detail below, the trainable XGBoost model preferably may be trained using the following hyperparameters in the following ranges: booster='gbtree'; colsample_bytree of from 0.9 to 1.0; gamma of from 0 to 0.1; max_depth of from 1 to 3; min_child_weight of from 9 to 11; and/or subsample of from 0.05 to 0.3; n_estimators of from 1000 to 1500; learning_rate of from 0.005 to 0.1; and/or scale_pos_weight of from 2 to 6. More preferably, the trainable XGBoost model is trained using the following hyperparameters: booster='gbtree'; colsample_bytree=1.0; gamma=0; max_depth=1; min_child_weight=10; subsample=0.1; n_estimators = 11652; learning_rate = 0.01; and/or scale_pos weight = 4.68.

As will also be outlined in further detail below, this set of hyperparameters is suited to provide training results leading to a trained XGBoost model on the basis of the allocated training data derived from the above-mentioned studies, the trained XGBoost model being capable of classifying the TCR reactivity with high reliability. For further details on these hyperparameters, as an example, reference may be made to the description of gradient boosting models in the XGBoost library, available e.g. under xgboost.readthedocs.io/en/stable/index.html. Alternatively, however, other libraries may be used, with comparable hyperparameters, as the skilled person will now, such as the Scikit-learn library.

The training method preferably may comprise splitting the allocated training data into a training data set and a test data set. Thus, more preferably, the splitting into a training data set and a test data set may a comprise x%-y% splitting of the allocated training data, with x being in the range 60 to 90, preferably in the range 65 to 75, and more preferably x= 70, and with y=100-x.

The training method may further comprise at least one cross-validation with differing splittings. Thus, a first validation may be performed with an x1%-y1% splitting of the allocated training data, and at least one second validation may be performed with an x2%-y2% splitting of the allocated training data, with x1≠x2, and the results of the validation may be compared. As an example, a degree of discrepancy between the results obtained for the different splittings may be used for qualifying and/or quantifying the training result.

Additionally or alternatively, the training method may further comprise repeatedly performing step (C) with differing splittings. Again, optionally, the training results, e.g. at least one numeric value qualifying and/or quantifying the training results, e.g. qualifying and/or quantifying the accuracy of the training, may be compared.

As outlined above, the training method preferably may comprise splitting the allocated training data into a training data set and a test data set. The training method may, then, further comprises the following step: at least one validation step, the validation step comprising testing the accuracy of the training by using the test data set.

The testing of the accuracy preferably may comprise using at least one model accuracy metrics. Various model accuracy metrics are generally known in the field of machine learning. Preferably, one or more or even all of the following model accuracy metrics may be used: AUC (area under the curve), overall accuracy, balanced accuracy, and/or weighted f1 score. Other options, however, are also feasible.

The training method may further comprise the following step: determining at least one target set of relevant genes, by using at least one feature extraction method.

Again, various feature extraction methods are generally known to the skilled person in the field of machine learning. As an example, the feature extraction method may comprise considering a permutation feature importance. Additionally or alternatively, the importance of the features may be quantified, such as by quantifying the influence a variation of the value of the respective feature has on the accuracy of the model. The feature extraction method may then, as an example, also comprise considering a feature importance hierarchy. Thus, as an example, the feature extraction method may comprise selecting all features having a feature importance of at least one threshold value. Additionally or alternatively, the feature extraction method may comprise selecting a predetermined number of features highest in ranking with respect to their feature importance. Thus, as an example, the feature extraction method may also comprise, additionally or alternatively, selecting a group of genes of the gene expression data of the T cells with highest TCR reactivity in the feature importance hierarchy to be the target set of relevant genes. Again, additionally or alternatively, the feature extraction method may also comprise selecting a predetermined number of genes of the gene expression data with the highest ranking in the feature importance hierarchy to be the target set of relevant genes. Other means and methods of feature extraction, from the training results, may also be used in addition or as an alternative. Preferably, feature extraction is performed using SHapley Additive exPlanations and feature selection based on SHAP value; corresponding methods are known in the art and have been reviewed e.g. in Lundberg & Lee, "A Unified Approach to Interpreting Model Predictions. in Advances in Neural Information Processing Systems" (eds. Guyon, I. et al.) vol. 30 (Curran Associates, Inc., 2017).

The feature extraction method may be performed such that a reasonable number of genes are selected to be the target set of relevant genes. As an example, for practical applications, selecting a predetermined number of from 5 to 500 genes is generally suitable for diagnostic handling, preferably a predetermined number of from 50 to 100 genes.

In a further aspect of the present invention, a computer-implemented classification method of classifying a TCR reactivity is disclosed. The TCR reactivity is selected from a predetermined group of at least two reactivity states, preferably as detailed herein above. The classification method comprises the following method steps, which, as an example, may be performed in the given order. However, a different order is also feasible. Further, it is possible to perform two or more of the method steps simultaneously or in a fashion overlapping in time. Further, it is also possible to perform one, more than one or even all of the method steps repeatedly.

The present invention also relates to a computer-implemented classification method comprising
(I) retrieving at least one trained model according to the method described herein above;
(II) retrieving gene expression data from T cells of said subject, the gene expression data comprising at least one predetermined target set of relevant genes; and
(II) applying the trained model to the gene expression data of step (II), thereby assigning a TCR to a reactivity class.

The aforesaid classification method may in particular be a computer-implemented method of predicting reactivity of a T cell receptor to target cells of a subject, so the present invention also relates to a computer-implemented method of predicting reactivity of a T cell receptor to target cells of a subject, the method comprising
(I) retrieving at least one trained model according to the method described herein above;
(II) retrieving gene expression data from T cells of said subject, the gene expression data comprising at least one predetermined target set of relevant genes; and
(III) applying the trained model to the gene expression data of step (II), thereby predicting reactivities of T cell receptors (TCRs) of said T cells to said target cells.

The trained model preferably may be trained by using the training method described herein, such as according to any one of the embodiments described above and/or according to any one of the embodiments described in further detail below. Thus, for most of the terms, definitions and options of the classification method, reference may be made to the training method as described above or as described in further detail below.

The group of predetermined reactivity states preferably is the same as discussed above in the context of the training method. The predetermined target set of relevant genes preferably comprises the target set of relevant genes determined during the training of the trained model. Thus, preferably, as discussed above, the trained model may be trained by using the training method of the present invention. As discussed above in the context of the training method, a target set of relevant genes may be determined during the training. This target set of relevant genes, in the classification method according to the present invention, may be used as the predetermined target set of relevant genes.

Step (III) further may comprise a step of optimization of class separation, e.g. by Jenks natural breaks optimization or Jenk-Fisher Break Optimisation. Also he classification method may further comprise the following step: (IV) outputting the assigned TCR reactivity state obtained in step (III).

The output, as an example, may take place in one or more of the following fashions: by electronic output, by data transfer, by data storage, by displaying, by acoustic output, by haptic output or by a combination of one or more or even all of the before-mentioned options, thus, as an example, the assigned reactivity state may be displayed on a computer display.

Also, the present invention relates to a system comprising a processor, said processor being configured for performing at least steps (B) and (C) of a method of training at least one trainable model and/or at least step (III) of the method of predicting reactivity of a T cell receptor, both methods as described herein above.

The system configured for performing at least steps (B) and (C) of a method of training at least one trainable model may also be referred to as a training system. The training system comprises at least one processor, the processor being configured, e.g. by software programming, for performing the training method described herein, such as according to any one of the embodiments of the training method described above and/or according to any one of the embodiments of the training method described in further detail below.

The system configured for performing at least step (III) of the method of predicting reactivity of a T cell receptor may also be referred to as a classification system. The classification system comprises at least one processor, the processor being configured, e.g. by software programming, for performing the classification method according to the present invention, such as according to any one of the embodiments of the classification method described above and/or according to any one of the embodiments of the classification method described in further detail below.

As the skilled person will understand in view of the description herein, the system may as well be c a combined training and classsification system.

The term "processor" is understood by the skilled person. The term preferably may refer, without limitation, to an arbitrary logic circuitry configured for performing basic operations of a computer or system, and/or, generally, to a device which is configured for performing calculations or logic operations. In particular, the processing unit may be configured for processing basic instructions that drive the computer or system. As an example, the processor may comprise at least one arithmetic logic unit (ALU), at least one floating-point unit (FPU), such as a math co-processor or a numeric coprocessor, a plurality of registers, preferably registers configured for supplying operands to the ALU and storing results of operations, and a memory, such as an L1 and L2 cache memory. In particular, the processor may be a multicore processor. Preferably, the processing unit is or comprises a central processing unit (CPU) and/or a graphics processing unit (GPU) . Additionally or alternatively, the processor may be or may comprise a microprocessor, thus preferably the processing unit's elements may be contained in one single integrated circuitry (IC) chip. Additionally or alternatively, the processing unit may be or may comprise one or more application-specific integrated circuits (ASICs) and/or one or more field-programmable gate arrays (FPGAs) or the like. The processing unit preferably may be configured, such as by software programming, for performing one or more evaluation operations.

The term "system" is understood by the skilled person. Preferably, the term includes each and every arbitrary set of interacting or interdependent components parts forming a whole. Preferably, the components interact with each other in order to fulfill at least one common function. The components may be handled independently or may be coupled or connectable. Consequently, the term "training system" generally refers to a system, as defined above, configured for performing at least one training method. Similarly, the term "classification system" generally relates to a system, as defined above, configured for performing at least one classification method.

In a further aspect of the present invention, a computer program is proposed, the computer program comprising instructions which, when the program is executed by at least one processor of a computer or a computer network cause the processor to perform the training method according to the present invention, such as according to any one of the embodiments of the training method described above and/or according to any one of the embodiments of the training method described in further detail below.

In a further aspect of the present invention, a computer program is proposed, the computer program comprising instructions which, when the program is executed by at least one processor of a computer or a computer network cause the processor to perform the classification method according to the present invention, such as according to any one of the embodiments of the classification method described above and/or according to any one of the embodiments of the classification method described in further detail below.

In a further aspect of the present invention, a computer-readable storage medium is proposed, preferably a non-transient computer-readable storage medium, the computer readable storage medium comprising instructions which, when the instructions are executed by at least one processor of a computer or a computer network cause the processor to perform the training method according to the present invention, such as such as according to any one of the embodiments of the training method described above and/or according to any one of the embodiments of the training method described in further detail below.

In a further aspect of the present invention, a computer-readable storage medium is proposed, preferably a non-transient computer-readable storage medium, comprising instructions which, when the instructions are executed by at least one processor of a computer or a computer network cause the processor to perform the classification method according to the present invention, such as according to any one of the embodiments of the classification method described above and/or according to any one of the embodiments of the classification method described in further detail below.

As outlined above, the methods as proposed herein are computer-implemented. As used herein, the terms "computer-readable data carrier" and "computer-readable storage medium" preferably may refer to non-transitory data storage means, such as a hardware storage medium having stored thereon computer-executable instructions. The computer-readable data carrier or storage medium preferably may be or may comprise a storage medium such as a random-access memory (RAM) and/or a read-only memory (ROM).

Thus, preferably, the method steps of the methods as indicated above may be performed by using a computer or a computer network, preferably by using a computer program.

Further disclosed and proposed herein is a computer program product having program code means, in order to perform any one of the methods according to the present invention in one or more of the embodiments enclosed herein when the program is executed on a computer or computer network. Preferably, the program code means may be stored on a computer-readable data carrier and/or on a computer-readable storage medium.

The methods and devices as proposed herein provide a large number of advantages over methods and devices of similar kind. Preferably, the above-mentioned technical challenges may be addressed, wherein, preferably, the accuracy of prediction may be increased. Further, as opposed to diagnosis based on single-platform gene expression data, the present invention also allows for combining different platforms from various studies. Thereby, larger and more diverse training data sets can be achieved which generally may lead to more appropriate and more precise machine learning models.

In view of the above, the following emboidments are particularly envisaged:
Embodiment 1: A method of identifying a T cell reactive to target cells of a subject presenting a T cell activating epitope (reactive T cell), comprising
   (a) determining expression of at least one biomarker selected from the group consisting of MAGEH1, HMGB2, TMSB10, MGAT4A, C12orf57, GIMAP7, HSPE1, STMN1, IL32, TPT1, MIF, PALM2-AKAP2, ISCU, CCR8, UGP2, EEF1B2, SETD7, MESD, LINC02099, IFT52, AKIRIN2, RIOK3, ANKRD12, CALR, DDX5, CNST, GNPDA1, MALAT1, RGL4, B2M, IFITM2, PTPN11, BTG1, ITSN2, RAD21, FYB1, DDX21, HSPD1, JAK1, ADAM19, TM2D3, CASP8, CARS1, PTPN7, HAPLN3, CSTB, TLE5, ACTG2, IL2RA, CD99, TMSB4X, CCR4, and PCM1 in T cells from a sample of said subject; and
   (b) identifying a reactive T cell based on the determination of step (a).
Embodiment 2: The method of embodiment 1, wherein step (a) further comprises determining expression of at least one biomarker selected from the group consisting of ABI3, AC243829.4, ACP5, AKAP13, ANXA1, APOBEC3G, AREG, CARHSP1, CCL3, CCNH, CD27, CD63, CD7, CHST12, CLIC3, CRIP1, CST7, CTLA4, CTSC, CXCL13, FOXP3, FTH1, GALNT2, GZMA, HLA-DRA, HMOX1, IFNG, IKZF3, IL6ST, IL7R, KIR2DL4, KLF6, LINC02446, LTB, LYAR, MIR4435-2HG, NKG7, PKM, PRF1, PSMB9, RABAC1, RBPJ, RGS1, RHOH, RSRP1, SPOCK2, and TOX.
Embodiment 3: A method of identifying a T cell reactive to cells of a subject presenting a T cell activating epitope (reactive T cell), comprising
   (a) determining expression of at least 10 biomarkers selected from the group consisting of CXCL13, AREG, MAGEH1, FOXP3, ANXA1, HMGB2, MIR4435-2HG, TMSB10, MGAT4A, C12orf57, GIMAP7, HSPE1, IL6ST, STMN1, AC243829.4, IL32, LINC02446, IFNG, NKG7, CTSC, TPT1, LYAR, MIF, PALM2-AKAP2, ISCU, CCR8, UGP2, EEF1B2, SETD7, LTB, CRIP1, APOBEC3G, RBPJ, RGS1, MESD, LINC02099, IKZF3, IFT52, AKIRIN2, RIOK3, CLIC3, ANKRD12, CALR, DDX5, CNST, GNPDA1, PKM, SPOCK2, RSRP1, IL7R, KIR2DL4, MALAT1, CTLA4, TOX, RGL4, KLF6, B2M, IFITM2, ABI3, CST7, PTPN11, BTG1, CCNH, ITSN2, RAD21, CCL3, HMOX1, FYB1, DDX21, GALNT2, HSPD1, GZMA, JAK1, CHST12, ADAM19, PRF1, CD63, TM2D3, CASP8, CARS1, ACP5, PTPN7, RHOH, HAPLN3, CSTB, TLE5, ACTG2, CD7, IL2RA, CD99, CARHSP1, TMSB4X, PSMB9, CCR4, AKAP13, RABAC1, HLA-DRA, FTH1, CD27, and PCM1, in T cells from a sample of said subject; and
   (b) identifying a reactive T cell based on the determination of step (a).
Embodiment 4: The method of any one of embodiments 1 to 3, wherein at least 20, preferably at least 30, more preferably at least 38, of said biomarkers are determined. Embodiment 5: The method of any one of embodiments 1 to 4, wherein at least 48 of said biomarkers are determined.
Embodiment 6: The method of any one of embodiments 1 to 5, wherein at most 1000, preferably at most 250, more preferably at most 100, even more preferably at most 50 biomarkers are determined.
Embodiment 7: The method of any one of embodiments 3 to 6, wherein said biomarkers comprise at least one biomarker selected from the group consisting of MAGEH1, HMGB2, MIR4435-2HG, TMSB10, MGAT4A, C12orf57, GIMAP7, HSPE1, IL6ST, STMN1, IL32, TPT1, LYAR, MIF, PALM2-AKAP2, ISCU, CCR8, UGP2, EEF1B2, SETD7, CRIP1, RGS1, MESD, LINC02099, IFT52, AKIRIN2, RIOK3, ANKRD12, CALR, DDX5, CNST, GNPDA1, SPOCK2, MALAT1, TOX, RGL4, B2M, IFITM2, ABI3, PTPN11, BTG1, ITSN2, RAD21, FYB1, DDX21, HSPD1, JAK1, ADAM19, TM2D3, CASP8, CARS1, PTPN7, HAPLN3, CSTB, TLE5, ACTG2, IL2RA, CD99, CARHSP1, TMSB4X, CCR4, and PCM1.
Embodiment 8: The method of any one of embodiments 1 to 7, wherein identifying a reactive T cell comprises determining a probability parameter for at least one T cell.
Embodiment 9: The method of any one of embodiments 1 to 8, wherein said probability parameter is indicative of the probability that said T cell is a reactive T cell.
Embodiment 10: The method of any one of embodiments 1 to 9, wherein step (b) comprises step (b1) comparing the result of the determining in step (a) to a reference.
Embodiment 11: The method of any one of embodiments 1 to 10, wherein expression is determined in step (a) by single-cell determination of gene expression, preferably by single-cell RNA sequencing.
Embodiment 12: The method of any one of embodiments 1 to 11, wherein said sample is a tumor sample.
Embodiment 13: The method of embodiment 12, wherein said T cell activating epitope is a tumor epitope, preferably a tumor specific epitope.
Embodiment 14: A method of identifying a TCR binding to an activating epitope presented on a cell, preferably a cancer cell, of a subject, said method comprising
   (A) identifying a reactive T cell according to the method of any one of embodiments 1 to 13,
   (B) providing the amino acid sequences of at least the complementarity determining regions (CDRs) 3 of the TCR of the reactive T cell identified in step (A); and, hereby,
   (C) identifying a TCR binding to an activating epitope presented on a cell.
Embodiment 15: The method of embodiment 14, wherein at least one biomarker of step (A) and/or the nucleic acid sequences in step (B) is/are determined by single-cell sequencing, preferably by single-cell RNA sequencing.
Embodiment 16: The method of embodiment 14 or 15, wherein said method comprises further step (B1) expressing a TCR comprising at least the CDRs determined in step (B) in a host cell, preferably a T cell.
Embodiment 17: The method of embodiment 16, wherein said method comprises further step (B2) determining binding of the TCR expressed in step (B1) to an activating epitope presented on a cell, preferably a cancer epitope, complexed in a major histocompatibility complex (MHC), preferably MHC class I, molecule.
Embodiment 18: The method of embodiment 16 or 17, wherein said method further comprises step (B3) determining recognition of cells presenting a T cell activating epitope by the TCR expressed in step (B1).
Embodiment 19: The method of any one of embodiments 14 to 18, wherein said method further comprises step (B4) producing a soluble TCR comprising at least the CDRs determined in step (B) and determining binding of said soluble TCR to a T cell activating epitope; preferably to a cancer epitope complexed in a major histocompatibility complex (MHC), preferably MHC class I, molecule.
Embodiment 20: A method of providing a T cell recognizing a cell presenting a T cell activating epitope, preferably a cancer cell, said method comprising
   (i) identifying a TCR binding to a cell presenting a T cell activating epitope according to the method according to any one of embodiments 14 to 19,
   (ii) expressing a TCR comprising at least the complementarity determining regions (CDRs) of the TCR of step (i) in a T cell, and, thereby,
   (iii) providing a T cell recognizing a cell presenting a T cell activating epitope, preferably a cancer cell.
Embodiment 21: The method of embodiment 20, wherein said method further comprises a step of testing reactivity of the T cell of step (ii) to cells presenting a T cell activating epitope.
Embodiment 22: The method of any one of embodiments 1 to 21, wherein said sample is a tissue sample or a bodily fluid sample.
Embodiment 23: The method of any one of embodiments 1 to 22, wherein said sample is a blood sample.
Embodiment 24: The method of any one of embodiments 1 to 22, wherein said sample is a cancer sample.
Embodiment 25: The method of any one of embodiments 1 to 23, wherein said sample is a sample of non-cancer tissue, preferably of cancer-adjacent tissue.
Embodiment 26: A reactive T cell identified by the method according to any one of embodiments 1 to 13 and/or obtained or obtainable by the method according to any one of embodiments 20 to 25.
Embodiment 27: The reactive T cell of embodiment 26 for use in medicine. Embodiment 28: The reactive T cell of embodiment 26 for use in treating and/or preventing cancer in a subject.
Embodiment 29: The subject matter of any one of embodiments 1 to 28, wherein said subject is an apparently healthy subject.
Embodiment 30: The subject matter of any one of embodiments 1 to 28, wherein said subject is a subject afflicted with cancer.
Embodiment 31: The subject matter of any one of embodiments 1 to 30, wherein said cells presenting a T cell activating epitope are cancer cells, preferably tumor cells.
Embodiment 32: The subject matter of any one of embodiments 1 to 31, wherein said TCR comprises, preferably consists of, a TCR alpha chain and a TCR beta chain or a TCR gamma chain and a TCR delta chain, preferably comprises, more preferably consists of, a TCR alpha chain and a TCR beta chain.
Embodiment 33: The subject matter of any of embodiments 1 to 32, wherein said reactive T cell is a cancer-reactive T cell.
Embodiment 34: The subject matter of any one of embodiments 1 to 33 wherein said T cell activating epitope is a cancer epitope, and wherein preferably said sample is a tumor sample.
Embodiment 35: The subject matter of any one of embodiments 1 to 34, wherein said sample is a sample of a brain metastasis of a non-brain primary tumor.
Embodiment 36: A polynucleotide encoding at least one TCR binding to an activating epitope provided or identified according to the method according to any one embodiments 14 to 19.
Embodiment 37: A device or kit comprising the reactive T cell according to any one of embodiments 20 to 25.
Embodiment 38: A method for treating and/or preventing cancer in a subject, said method comprising
   (a) administering a reactive T cell according to embodiment 26 to said subject; and
   (b) thereby treating and/or preventing cancer in said subject.
Embodiment 39: A method for treating and/or preventing cancer in a subject, said method comprising
   (A) providing or having provided a T cell recognizing a cell presenting a T cell activating epitope according embodiment 26;
   (B) administering the reactive T cell of step (A) to said subject; and
   (C) thereby treating and/or preventing cancer in said subject.
Embodiment 40: Use of a method of any one of embodiments 1 to 25 and/or a reactive T cell according to embodiment 26 for providing a medicament, wherein said medicament preferably is for use in treating cancer.
Embodiment 41: A computer-implemented method of training at least one trainable model for identifying a T cell reactive to target cells of a subject presenting a T cell activating epitope (reactive T cell), the method comprising:
   (A) providing the trainable model;
   (B) retrieving allocated training data, the allocated training data comprising gene expression data of T cells allocated to reactivities of the T cell receptors (TCRs) of said T cells to said target cells, wherein said gene expression data and said reactivities of the TCRs were obtained by the method according to any one of embodiments 1 to 13; and
   (C) training the trainable model on the allocated training data.
Embodiment 42: A computer-implemented method of providing a trained model for identifying a reactive T cell, said method comprising the steps of the method of embodiment 41, and further step (D) thereby providing a trained model for identifying a reactive T cell.
Embodiment 43: The method of embodiment 41 or 42, wherein the trainable model is selected from a trainable XGBoost model, a trainable Adaboost model, a trainable Decision Tree model, a trainable Catboost model, a trainable Gaussian Naive Bayes model, a trainable K-nearest neighbour model, a trainable Logistic regression model, a trainable Radial Basis Function (RBF) Support Vector Machine (SVM) model, a trainable Random Forest model, and a trainable linear SVM model.
Embodiment 44: The method of any one of embodiments 41 to 43, wherein the trainable model is a trainable XGBoost model.
Embodiment 45: The method of any one of embodiments 41 to 44, wherein the reactivities of the TCRs are relative reactivity values compared to the overall reactivities of said T cells, and/or are relative reactivities compared to a reference.
Embodiment 46: The method of any one of embodiments 41 to 45, wherein the gene expression data of the allocated training data in step (B) are derived from tumor-infiltrating lymphocytes and are, preferably, obtained by mRNA analysis.
Embodiment 47: The method of any one of embodiments 41 to 46, wherein the training method comprises at least one hyperparameter tuning step for tuning hyperparameters of the trainable model.
Embodiment 48: The method of embodiment 47, wherein said hyperparameter tuning comprises tuning of parameter colsample_bytree, gamma, learning_rate, max_delta_step, max_depth, min_child_weight, n_estimators, alpha, lambda, scale_pos weight, and/or subsample.
Embodiment 49: The method of embodiment 47 or 48, wherein said hyperparameter tuning comprises Bayesian optimization using scikit-optimize with 10 stratified k-fold cross validation.
Embodiment 50: The method of any one of embodiments 41 to 49, wherein said method comprises a step of feature selection based on Shapley additive explanation (SHAP) values and repetition of hyperparameter tuning using the selected features.
Embodiment 51: The method of any one of embodiments 41 to 50, wherein the trainable model comprises a trainable XGBoost model, wherein the trainable XGBoost model is trained using the following hyperparameters in the following ranges: booster='gbtree'; colsample_bytree of from 0.9 to 1.0; gamma of from 0 to 0.1; max_depth of from 1 to 3; min_child_weight of from 9 to 11; and/or subsample of from 0.05 to 0.3; n_estimators of from 1000 to 1500; learning_rate of from 0.005 to 0.1; and/or scale_pos_weight of from 2 to 6.
Embodiment 52: The method of any one of embodiments 41 to 51, wherein the allocated training data are data obtained from a plurality of different subjects.
Embodiment 53: The method of any one of embodiments 41 to 52, wherein the allocated training data additionally contain information on T cell subtype and/or exhaustion markers.
Embodiment 54: The method of any one of embodiments 41 to 53, wherein the training comprises splitting the allocated training data into a training data set and a test data set.
Embodiment 55: The method of any one of embodiments 41 to 54, wherein the method comprises a cross-validation with differing splittings.
Embodiment 56: The method of any one of embodiments 41 to 55, wherein the method further comprises at least one validation step, the validation step comprising testing the accuracy of the training by using the test data set or using an independent data set.
Embodiment 57: The method of any one of embodiments 41 to 56, wherein the method further comprises determining at least one target set of relevant genes, by using at least one feature extraction method.
Embodiment 58: The method according to embodiment 57, wherein the feature extraction method comprises considering a permutation feature importance.
Embodiment 59: The method of embodiment 57 or 58, wherein the feature extraction method comprises considering SHapley Additive exPlanations (SHAP) and feature selection based on SHAP value.
Embodiment 60: The method of any one of embodiments 41 to 59, wherein the allocated training data comprise data obtained from T cells of at least one apparently healthy subject.
Embodiment 61: The method of any one of embodiments 41 to 60, wherein the allocated training data comprise data obtained from T cells of a plurality of apparently healthy subjects.
Embodiment 62: The method of any one of embodiments 41 to 61, wherein in the allocated training data the number of T cells from apparently healthy subjects represented is at least 5fold, preferably at least 10fold, more preferably at least 25 fold, even more preferably at least 50fold, most preferably at least 75 fold, the number of TILs represented.
Embodiment 63: A computer-implemented classification method comprising
   (a) retrieving at least one trained model according to the method according to any one of embodiments 43 to 70;
   (b) retrieving gene expression data from T cells of said subject, the gene expression data comprising at least one predetermined target set of relevant genes; and
   (c) applying the trained model to the gene expression data of step (II), thereby assigning a TCR to a reactivity class.
Embodiment 64: A computer-implemented method of predicting reactivity of a T cell receptor to target cells of a subject, the method comprising
   (I) retrieving at least one trained model according to the method according to any one of embodiments 41 to 62;
   (II) retrieving gene expression data from T cells of said subject, the gene expression data comprising at least one predetermined target set of relevant genes; and
   (III) applying the trained model to the gene expression data of step (II), thereby predicting reactivities of T cell receptors (TCRs) of said T cells to said target cells.
Embodiment 65: The method of embodiment 63 or 64, wherein the trained model is the trained model obtained according to any one of embodiments 41 to 62.
Embodiment 66: The method of embodiment 64 or 65, further comprising step (IV) outputting the predicted reactivities for said TCRs.
Embodiment 67: The method of embodiment 66, wherein said reactivities are probabilities of being reactive.
Embodiment 68: A system comprising a processor, said processor being configured for performing at least steps (B) and (C) of a method according to any one of embodiments 41 to 62 and/or at least step (III) of the method according to any one of embodiments 63 to 67.
Embodiment 69: The system of embodiment 68 comprising, preferably tangibly embedded, an executable code which, when executed, causes the system to perform at least steps (B) and (C) of a method according to any one of embodiments 41 to 62 and/or at least step (III) of the method according to any one of embodiments 63 to 67.
Embodiment 70: The system of embodiment 68 or 69, further comprising a data storage unit comprising the allocated training data.
Embodiment 71: The system of embodiment 68 or 70, further comprising a code storage unit comprising said executable code.
Embodiment 72: A trained model obtained or obtainable by a method according to any one of embodiments 41 to 62, preferably tangibly embedded on a data carrier.
Embodiment 73: A method for treating cancer in a patient, said method comprising
   (a) identifying at least one reactive TCR in a sample of said subject with a model trained according to any one of embodiments 41 to 62,
   (b) providing or having provided T cells expressing the at least one TCR identified in step (a);
   (c) administering the T cells of step (ii) to the subject, and thereby
   (d) treating said cancer in said subject.
Embodiment 74: The method of embodiment 73, wherein said sample is a tumor sample.
Embodiment 75: Use of a trained model according to embodiment 72 for identifying at least one reactive TCR.
Embodiment 76: A computer program comprising instructions which, when the program is executed by at least one processor of a computer or a computer network cause the processor to perform the method according to any one of embodiments 41 to 62 and/or at least step (III) of the method according to any one of embodiments 63 to 67.
Embodiment 77: A computer-readable storage medium, specifically a non-transient computer-readable storage medium, comprising instructions which, when the instructions are executed by at least one processor of a computer or a computer network cause the processor to perform the method according to any one of embodiments 41 to 62 and/or at least step (III) of the method according to any one of embodiments 63 to 67.

All references cited in this specification are herewith incorporated by reference with respect to their entire disclosure content and the disclosure content specifically mentioned in this specification.

### Figure Legends

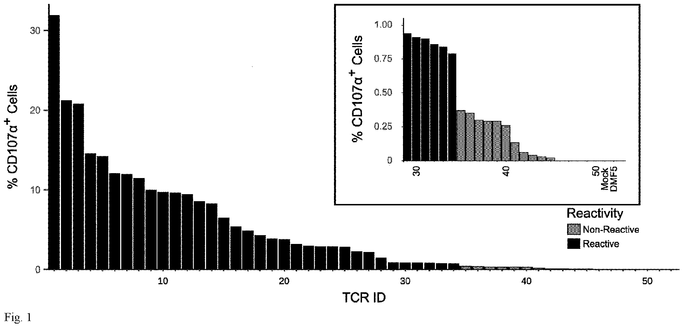

Fig. 1: Top 50 TCR clones of patient BT21 were co-cultured with BT21 patient-derived cell lines and the expression of CD107a was measured by flow cytometry with mock and DMF5 TCR included as negative control. Insert: enlarged representation for TCR IDs 29-50 and negative controls.

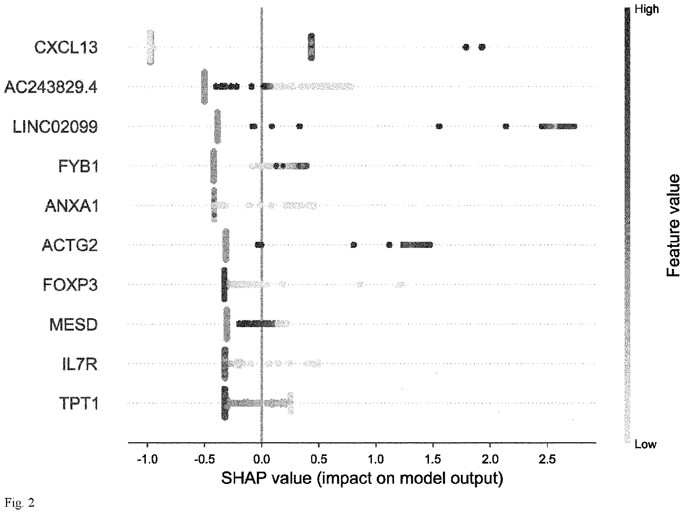

Fig. 2: SHapley Additive exPlanation (SHAP) analysis performed on predicTCR. The top 10 genes were illustrated with beeswarm plot, with each dot representing a single cell. The greyscale represents the expression and x-axis represent the SHAP value, in which a positive value contributes to reactive prediction and vice versa. Thus, e.g. for TPT1, high expression is predictive of non-reactivity, and low expression is predictive of reactivity.

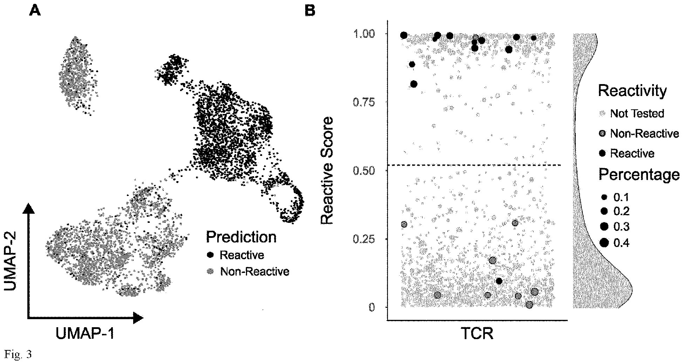

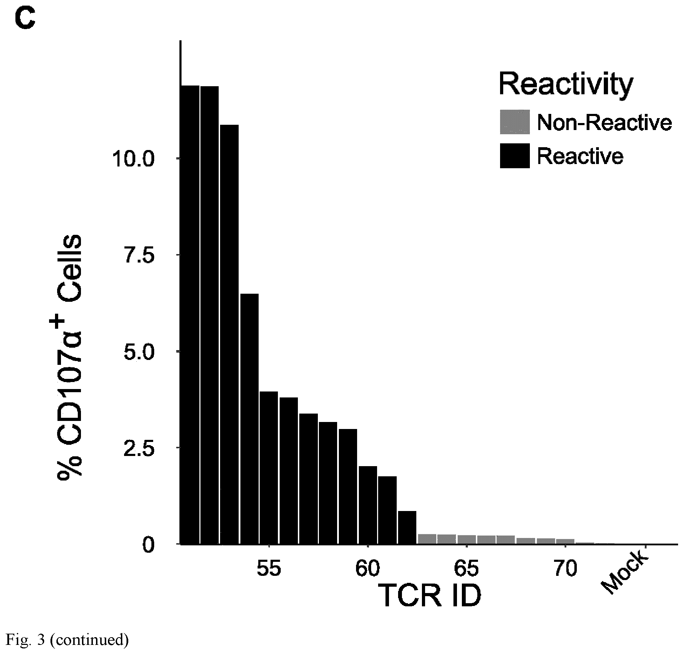

Fig. 3: (A) The prediction of reactivity on all BT21 T Cells projected on a UMAP plot. **(B)** The reactivity score for each TCR was determined from individual T cells using probability of reactivity, as assessed by the classifier. The density plot show the distribution of TCR reactivity score and dotted line indicates threshold calculated using Fisher-Jenk natural break optimization. **(C)** Based on (B), a further 22 TCRs from BT21 patient were tested by co-culturing with patient-derived cell lines. CD107a was measured similarly with flow cytometry.

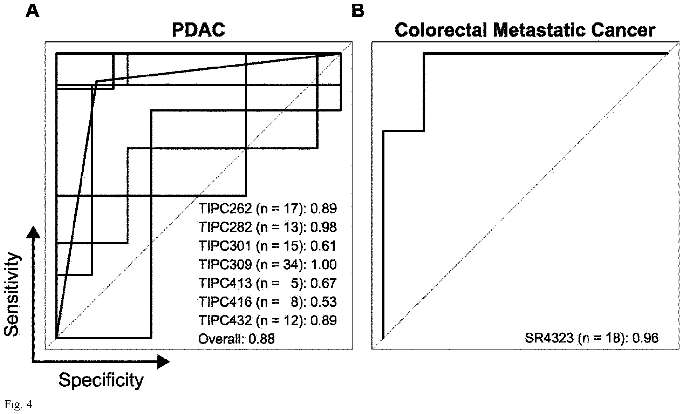

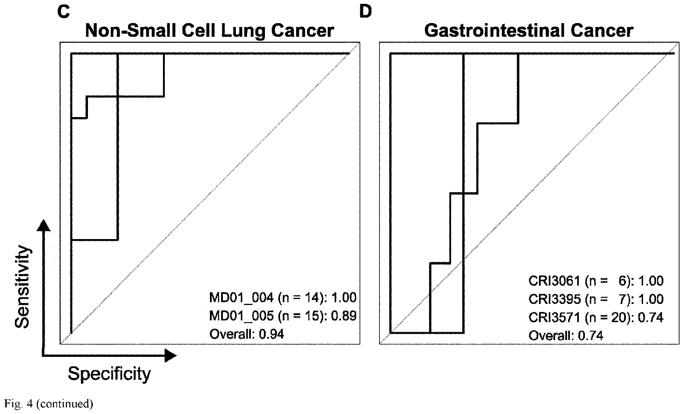

Fig. 4: The performance of predicTCR as shown by AUC plots on **(A)** PDAC, **(B)** Colorectal Metastatic Cancer, **(C)** Non-Small Cell Lung Cancer and **(D)** Gastrointestinal Cancer. See Table 3 for comprehensible metrics. Codes are patient codes of Table 3.

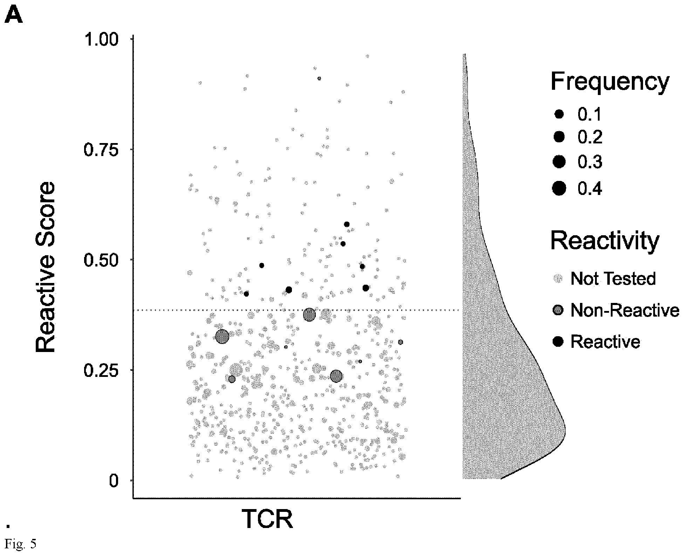

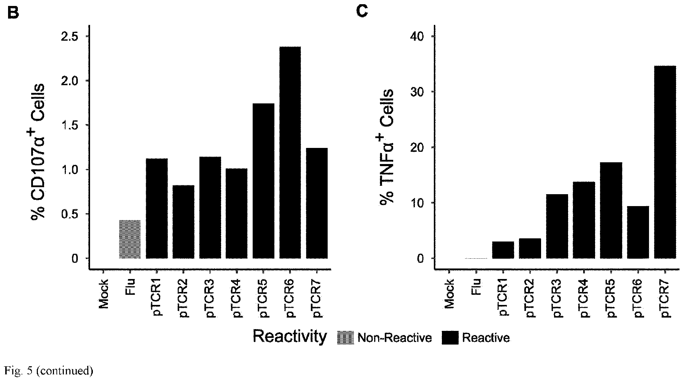

Fig. 5: (A) Prediction of tumor reactive TCR using predicTCR in the prospective patient of Example 6.4. The density plot show the distribution of TCR reactivity score and the dotted line indicates the prediction threshold calculated using Fisher-Jenk natural break optimization. Reactivity test of predicted TCRs measured by **(B)** CD107a and **(C)** TNFα. Mock and flu TCR were included as control.

The following Examples shall merely illustrate the invention. They shall not be construed, whatsoever, to limit the scope of the invention. Exemplary protocols for performing the Examples below can also be derived from the Literature indicated below, in particular Meng et al., Sci. Transl. Med. 15, eadh9562 (2023), Lowery et al., Science 375, 877-884 (2022), Caushi. et al., Nature 596, 126-132 (2021), and Zheng et al. Cancer Cell 40, 410- 423.e7 (2022).

### Example 1: Single Cell Library Preparation

Single cell tumor suspension was FACS-sorted for CD45⁺CD3⁺ population to enrich for T cells. Single Cell library of sorted T cells was prepared using 10X Single Cell Immune Profiling Kit according to manufacturer's protocols. The resulting scVDJ and scRNA library were then sequenced on Novaseq-6k.

### Example 2: Single Cell RNA Analysis

Raw data was processed with cellranger pipeline (v6.1.2) to generate gene expression matrices. Matrices were imported into Rand analyzed using Seurat. For quality control, SoupX was used to remove background noise and miQC to removed cells with high mitochondrial gene expression. Additionally, outliers were also removed based on UMI and the number of genes. The data was then normalized using sctransform method.

### Example 3: Co-culture Assay

RNA for each individual TCR was prepared using Cellscript kit according to manufacturer's protocol. The concentration and integrity of RNA was assessed by Nanodrop and Tapestation respectively. The TCRs were then expressed by electroporation of RNA into expanded PBMC using the Lonza 4D nucleofactor device. The cells were then plated into 48-well plates containing TexMACS media supplemented with 2% AB serum and allowed to rest overnight. The electroporated cells were then co-cultured with target cells (patient-derived tumor cell line) with CD107a FACS antibody added for 5 hours, with the addition of Golgistop and Golgiplug after 1 hour. After co-culture, the cells were stained for DCM (dead cell marker), CD3, CD4, CD8a, mTCRβ and IFNγ,and then measured using FACSlyrics (BD Biosciences). Further analysis of data was done using FlowJo.

### Example 4: predicTCR Classifier Training

The normalized transcriptomic data of tested BT21 TCR (1461 cells) was used as training data input, with additional data from healthy donor PBMC (112960 cells). Healthy PBMC datasets were obtained from multiple sources to increase variability: a single donor from 10X Genomics (www.10xgenomics.com/resources/datasets/human- T cells-from-a-healthy-donor-1-k-cells-multi-v-2-2-standard-5-0-0), two donors from Szabo et al., and seven donors from Gao et al. TCR reactivity (label) based on CD107a expression in co-culture assay was converted into binary value and all healthy donor PBMCs were assumed to be non-reactive. Hyperparameter tuning was performed using Bayesian optimization (scikit-optimize) with 10 stratified k-fold cross validation for XGBoost hyperparameters 'colsample_bytree', 'gamma', 'learning_rate', 'max_delta_step', 'max_depth', 'min_child_weight', 'n_estimators', 'alpha', 'lambda', 'scale_pos_weight' and 'subsample'. To prevent overfitting, a simpler model was trained by identification key genes contributing to the model using Shapley Additive exPlanations (SHAP). The final classifier (termed predicTCR) was then trained based on the top 100 SHAP features (genes) and hyperparameters were optimized as before.

### Example 5: Prediction of TCR using predicTCR

All raw data for validation of predicTCR was processed as described above. The probability of reactivity, as predicted by predicTCR, was average for each TCR clonotype (Reactive Score, which may also be referred to as "Reactivity Score"). Using Fisher-Jenks natural break optimization, the threshold for distinguishing reactive and non-reactive TCR clonotypes was determined.

### Example 6: Results

### 6.1 Proof of Principle for ML-based approach

To train a machine learning(ML) based model for prediction of tumor reactive TCR, we have cloned and tested the top 50 TCRs from a melanoma brain metastasis patient (BT21) in unbiased fashion. The reactivity of TCR was then determined using co-culture assay (Figure 1). The transcriptome data was then used for training with the reactivity as label. Using various machine learning models, ML-based approach perform well (AUC > 0.85); AUC values determined were for the respective models (model: AUC): Adaboost: 0.90; Decision Tree: 0.89; Catboost: 0.90; Gaussian Naive Bayes: 0.90; K-nearest Neighbor: 0.85; Logistic Regression: 0.85; SVM (RBF): 0.86; Random Forest: 0.88; SVM (Linear): 0.86; and XGBoost: 0.92; thus, XGBoost performed best (AUC = 0.92). To train a simpler model, SHAP analysis was performed and only the top 100 genes identified via SHAP was included in retraining. The top 10 genes were identified by SHapley Additive exPlanation (SHAP) analysis(Figure 2). As shown in Figure 2, there is not in each case a direct proportionality between the expression level of a biomarker and impact on model output in the model. Without wishing to be bound by theory, it is assumed that this may be caused by non-linear contributions of biomarkers to the model (e.g. contribution only starting from and/or up to a given threshold value), and/or mutual interdependencies between biomarkers (contribution of marker A only if marker B is present at a given level).

### 6.2 Prospective Prediction of Tumor Reactive TCR

Using XGBoost, a classifier was trained using the 50 TCRs with additional PBMC healthy datasets (Szabo et al. Nat. Commun. 10:4706 (2019); Gao et al., Nat. Commun. 13:1982 (2022)) and the hyperparameters was determined using Bayesian optimization. The prediction was then performed on all T cells from BT21 (Figure 3A). A further 22 TCRs were selected based on the prediction (Figure 3B) and tested with co-culture assay (Figure 3C). 20 out of 22 (91% accuracy) of the TCR were predicted correctly (Figure 3 B,C), which is higher than all other signature-based approaches (Table 2).

### 6.3 Prediction of Tumor Reactive TCR from Diverse Tumor Type

To determine the generalizability of TCR, the performance of predicTCR was evaluated using external datasets from various diverse tumor types (Table 3). When applied to pancreatic ductal adenocarcinoma, predicTCR was able to predict tumor reactive TCR well with Overall AUC of 0.88 (Figure 4A). Similarly, when applied to colorectal metastatic cancer and non-small cell lung cancer, predicTCR was also able to predict tumor-reactive TCR with AUC of 0.96 and 0.94 respectively (Figure 4B,C). Even when extending predicTCR to single cell data generated using an other technology such as smart-seq, predicTCR was also able to predict tumor-reactive TCR from gastrointestinal cancer, with AUC of 0.74. Furthermore, predicTCR performed better than all other signature based approaches (see Tables 4-7 for detailed comparisons).

### 6.4 Prospective Prediction of Tumor Reactive TCR in New Patient

In a patient (TIPC418) of *Meng* et al., all of the 8 predicted reactive TCRs tested negative. However, predicTCR was able to identify many potential reactive TCRs (Figure 5A). Hence, based on predicTCR, we further cloned 7 TCRs from this patient and tested them with the patient-derived tumor cell line with co-culture assay. Based on CD107a and TNFα expression in flow cytometry, all 7 of the predicted TCRs were indeed reactive, further showcasing the predictability of predicTCR (Figure 5B,C).

### Literature

- Bernardeau et al., (2011), J Immunol Methods, 371(1- 2):97- 105
- Bordner (2010), PLoS ONE 5(12): e14383
- Cano- Gamez et al., Nat Comm 11:, art. 1801 (2020) (doi.org/10.1038/s41467- 020- 15543-y)
- Caushi. et al., Nature 596, 126-132 (2021)
- Crompton et al., Immunol. Rev. 257, 264-276 (2014)
- Gao et al., Nat. Commun. 13:1982 (2022)
- Lowery et al., Science 375, 877-884 (2022)
- Lundberg & Lee, "A Unified Approach to Interpreting Model Predictions. in Advances in Neural Information Processing Systems" (eds. Guyon, I. et al.) vol. 30 (Curran Associates, Inc., 2017)
- Magen et al., Cell Rep 29(10):3019 (2019)
- Meng et al., Sci. Transl. Med. 15, eadh9562 (2023)
- Nielsen et al., (2004), Bioinformatics, 20 (9), 1388-1397
- Oh et al., Cell 181(7):1612 (2020)
- Poschke et al., Clin. Cancer Res. 26, 4289-4301 (2020)
- Rohaan et al., N. Engl. J. Med. 387, 2113-2125 (2022)
- Simoni et al., Nature 557, 575-579 (2018)
- Szabo et al. Nat. Commun. 10:4706 (2019)
- WO2018/209324
- WO2019/070755
- WO 2021/188954 A1
- WO 2022/200456 A1
- WO 2022/200457 A1
- Zheng et al. Cancer Cell 40, 410- 423.e7 (2022)

**Table 1: Preferred biomarkers, their full gene names, and database accession numbers. For AC243829.4, also the designation CCL3-AS1 has been used.**

| No. | Gene Name | Full Gene Name | Ensembl ID | Entrez ID | Refseq ID |
|---|---|---|---|---|---|
| 1 | CXCL13 | C-X-C motif chemokine ligand 13 | ENSG00000156234 | 10563 | NM_006419.3; NM_001371558.1 |
| 2 | AREG | amphiregulin | ENSG00000109321 | 374 | NM_001657.4 |
| 3 | MAGEH1 | MAGE family member H1 | ENSG00000187601 | 28986 | NM_014061.5 |
| 4 | FOXP3 | forkhead box P3 | ENSG00000049768 | 50943 | NM_014009.4; NM_001114377.2 |
| 5 | ANXA1 | annexin A1 | ENSG00000135046 | 301 | NM_000700.3 |
| 6 | HMGB2 | high mobility group box 2 | ENSG00000164104 | 3148 | NM_002129.4; NM_001130688.1; NM_001130689.1 |
| 7 | MIR4435-2HG | MIR4435-2 host gene | ENSG00000172965 | 541471 | NR_015395.2; NR_024373.2; NR_136161.1; NR_136162.1; NR_136163.1; NR_136164.1; NR_136165.1; NR_136166.1 |
| 8 | TMSB10 | thymosin beta 10 | ENSG00000034510 | 9168 | NM_021103.4 |
| 9 | MGAT4A | alpha-1,3-mannosyl-glycoprotein 4-beta-N-acetylglucosaminyltransferase A | ENSG00000071073 | 11320 | NM_012214.3; NM_001160154.2 |
| 10 | C12orf57 | chromosome 12 open reading frame 57 | ENSG00000111678 | 113246 | NM_138425.4; NM_001301834.1; NM_001301836.2; NM_001301837.2; NM_001301838.2 |
| 11 | GIMAP7 | GTPase; IMAP family member 7 | ENSG00000179144 | 168537 | N M_1 53236.4 |
| 12 | HSPE1 | heat shock protein family E (Hsp10) member 1 | ENSG00000115541 | 3336 | NM_002157.3 |
| 13 | IL6ST | interleukin 6 cytokine family signal transducer | ENSG00000134352 | 3572 | NM_002184.4; NM_175767.3; NM_001190981.2; NM_001364275.2; NM_001364276.2; NM_001364277.2; NM_001364278.2; NM_001364279.2 |
| 14 | STMN1 | stathmin 1 | ENSG00000117632 | 3925 | NM_203401.2; NM_203399.2; NM_005563.4; NM_001145454.3 |
| 15 | AC243829.4 (CCL3-AS1) | CCL3 Antisense RNA 1 | ENSG00000277089 | 10272485 0 | NR_186417.1 |
| 16 | IL32 | interleukin 32 | ENSG00000008517 | 9235 | NM_001012631.4; NM_004221.7; NM_001012632.3; NM_001012633.4; NM_001012634.4; NM_001012635.4; NM_001012636.2; NM_001012718.4; NM_001308078.4; NM_001369587.3; NM_001369588.3; NM_001369589.3; NM_001369590.3; NM_001369591.3; NM_001369592.3; NM_001369593.3; NM_001369595.3; NM_001369596.3; NM_001376923.1 |
| 17 | LINC02446 | long intergenic non-protein coding RNA 2446 | ENSG00000256039 | 10106003 8 | NR_146455.1; NR_146456.1 |
| 18 | IFNG | interferon gamma | ENSG00000111537 | 3458 | NM_000619.3 |
| 19 | NKG7 | natural killer cell granule protein 7 | ENSG00000105374 | 4818 | NM_005601.4; NM_001363693.2 |
| 20 | CTSC | cathepsin C | ENSG00000109861 | 1075 | NM_001814.6; NM_148170.5; NM_001114173.3 |
| 21 | TPT1 | tumor protein; translationally-controlled 1 | ENSG00000133112 | 7178 | NM_001286272.2; NM_003295.4; NM_001286273.2 |
| 22 | LYAR | Ly1 antibody reactive | ENSG00000145220 | 55646 | NM_017816.3; NM_001145725.2 |
| 23 | MIF | macrophage migration inhibitory factor | ENSG00000240972 | 4282 | NM_002415.2 |
| 24 | PALM2-AKAP2 | PALM2 and AKAP2 fusion | ENSG00000157654 | 445815 | NM_053016.6; NM_001037293.3; NM_007203.5; NM_147150.3; NM_001004065.4; NM_001198656.1; NM_001136562.3 |
| 25 | ISCU | iron-sulfur cluster assembly enzyme | ENSG00000136003 | 23479 | NM_014301.4; NM_213595.4; NM_001301140.1; NM_001301141.1; NM_001320042.1 |
| 26 | CCR8 | C-C motif chemokine receptor 8 | ENSG00000179934 | 1237 | NM_005201.4 |
| 27 | UGP2 | UDP-glucose pyrophosphorylase 2 | ENSG00000169764 | 7360 | NM_006759.4; NM_001001521.2; NM_001377524.1; NM_001377525.1; NM_001377526.1; NM_001377527.1; NM_001377528.1; NM_001377529.1 |
| 28 | EEF1B2 | eukaryotic translation elongation factor 1 beta 2 | ENSG00000114942 | 1933 | NM_001959.4; NM_021121.4; NM_001037663.2 |
| 29 | SETD7 | SET domain containing 7; histone lysine methyltransferase | ENSG00000145391 | 80854 | NM_030648.4; NM_001306199.2; NM_001306200.2 |
| 30 | LTB | lymphotoxin beta | ENSG00000227507 | 4050 | NM_002341.2; NM_009588.1 |
| 31 | CRIP1 | cysteine rich protein 1 | ENSG00000213145 | 1396 | NM_001311.5 |
| 32 | APOBEC3G | apolipoprotein B mRNA editing enzyme catalytic subunit 3G | ENSG00000239713 | 60489 | NM_021822.4; NM_001349436.1; NM_001349437.2; NM_001349438.3 |
| 33 | RBPJ | recombination signal binding protein for immunoglobulin kappa J region | ENSG00000168214 | 3516 | NM_005349.4; NM_015874.6; NM_203283.5; NM_203284.3; NM_001363577.2; NM_001374400.1; NM_001374401.1; NM_001374402.1; NM_001374403.1; NM_001379406.1; NM_001379407.1; NM_001379408.1; NM_001379409.1 |
| 34 | RGS1 | regulator of G protein signaling 1 | ENSG00000090104 | 5996 | NM_002922.4 |
| 35 | MESD | mesoderm development LRP chaperone | ENSG00000117899 | 23184 | NM_015154.3 |
| 36 | LINC02099 | long intergenic non-protein coding RNA 2099 | ENSG00000253490 | 10192945 0 | NR_125814.1; NR_125815.1; NR_125816.1 |
| 37 | IKZF3 | IKAROS family zinc finger 3 | ENSG00000161405 | 22806 | NM_012481.5; NM_183228.3; NM_183229.3; NM_183230.3; NM_183231.3; NM_183232.3; NM_001257408.2; NM_001257409.2; NM_001257410.2; NM_001257411.2; NM_001257412.2; NM_001257413.2; NM_001257414.2; NM_001284514.2; NM_001284515.2; NM_001284516.1 |
| 38 | IFT52 | intraflagellar transport 52 | ENSG00000101052 | 51098 | NM_016004.5; NM_001303458.3; NM_001303459.3; NM_001323578.2; NM_001323579.2; NM_001323580.2; NM_001323581.2 |
| 39 | AKIRIN2 | akirin 2 | ENSG00000135334 | 55122 | NM_018064.4 |
| 40 | RIOK3 | RIO kinase 3 | ENSG00000101782 | 8780 | NM_003831.5; NM_001348193.2 |
| 41 | CLIC3 | chloride intracellular channel 3 | ENSG00000169583 | 9022 | NM_004669.3 |
| 42 | ANKRD12 | ankyrin repeat domain 12 | ENSG00000101745 | 23253 | NM_015208.5; NM_001083625.3; NM_001204056.1 |
| 43 | CALR | calreticulin | ENSG00000179218 | 811 | NM_004343.4 |
| 44 | DDX5 | DEAD-box helicase 5 | ENSG00000108654 | 1655 | NM_001320595.2; NM_004396.5; NM_001320596.3; NM_001320597.2 |
| 45 | CNST | consortin; connexin sorting protein | ENSG00000162852 | 163882 | NM_152609.3; NM_001139459.2 |
| 46 | GNPDA1 | glucosamine-6-phosphate deaminase 1 | ENSG00000113552 | 10007 | NM_005471.5 |
| 47 | PKM | pyruvate kinase M1/2 | ENSG00000067225 | 5315 | NM_002654.6; NM_182470.4; NM_182471.4; NM_001206796.3; NM_001206797.3; NM_001206798.3; NM_001206799.2; NM_001316318.2; NM_001411081.1 |
| 48 | SPOCK2 | SPARC (osteonectin); cwcv and kazal like domains proteoglycan 2 | ENSG00000107742 | 9806 | NM_001134434.1; NM_014767.2; NM_001244950.2 |
| 49 | RSRP1 | arginine and serine rich protein 1 | ENSG00000117616 | 57035 | NM_020317.5; NM_001321772.2 |
| 50 | IL7R | interleukin 7 receptor | ENSG00000168685 | 3575 | NM_002185.5; NM_001410734.1 |
| 51 | KIR2DL4 | killer cell immunoglobulin like receptor; two Ig domains and long cytoplasmic tail 4 | ENSG00000189013 | 3805 | NM_002255.6; NM_001080772.2; NM_001080770.2 |
| 52 | MALAT1 | metastasis associated lung adenocarcinoma transcript 1 | ENSG00000251562 | 378938 | NR_002819.4; NR_144567.1; NR_144568.1 |
| 53 | CTLA4 | cytotoxic T-lymphocyte associated protein 4 | ENSG00000163599 | 1493 | NM_005214.5; NM_001037631.3 |
| 54 | TOX | thymocyte selection associated high mobility group box | ENSG00000198846 | 9760 | NM_014729.3 |
| 55 | RGL4 | ral guanine nucleotide dissociation stimulator like 4 | ENSG00000159496 | 266747 | NM_001329424.3; NM_153615.2; NM_001329425.2 |
| 56 | KLF6 | KLF transcription factor 6 | ENSG00000067082 | 1316 | NM_001300.6; NM_001160124.2; NM_001160125.2 |
| 57 | B2M | beta-2-microglobulin | ENSG00000166710 | 567 | NM_004048.4 |
| 58 | IFITM2 | interferon induced transmembrane protein 2 | ENSG00000185201 | 10581 | NM_006435.3 |
| 59 | ABI3 | ABI family member 3 | ENSG00000108798 | 51225 | NM_001135186.2; NM_016428.3 |
| 60 | CST7 | cystatin F | ENSG00000077984 | 8530 | NM_003650.4 |
| 61 | PTPN11 | protein tyrosine phosphatase non-receptor type 11 | ENSG00000179295 | 5781 | NM_002834.5; NM_080601.3; NM_001330437.2; NM_001374625.1 |
| 62 | BTG1 | BTG anti-proliferation factor 1 | ENSG00000133639 | 694 | NM_001731.3 |
| 63 | CCNH | cyclin H | ENSG00000134480 | 902 | NM_001239.4; NM_001199189.2; NM_001363539.2; NM_001364075.2; NM_001364076.2 |
| 64 | ITSN2 | intersectin 2 | ENSG00000198399 | 50618 | NM_006277.3; NM_147152.3; NM_019595.4; NM_001348181.2; NM_001348182.2; NM_001348183.2; NM_001348184.2; NM_001348185.2; NM_001348186.2 |
| 65 | RAD21 | RAD21 cohesin complex component | ENSG00000164754 | 5885 | NM_006265.3 |
| 66 | CCL3 | C-C motif chemokine ligand 3 | ENSG00000277632 | 6348 | NM_002983.3 |
| 67 | HMOX1 | heme oxygenase 1 | ENSG00000100292 | 3162 | NM_002133.3 |
| 68 | FYB1 | FYN binding protein 1 | ENSG00000082074 | 2533 | NM_001465.6; NM_199335.5; NM_001243093.2; NM_001349333.2; NM_018594.2 |
| 69 | DDX21 | DExD-box helicase 21 | ENSG00000165732 | 9188 | NM_004728.4; NM_001256910.2; NM_001410932.1 |
| 70 | GALNT2 | polypeptide N-acetylgalactosaminyltransferase 2 | ENSG00000143641 | 2590 | NM_004481.5; NM_001291866.2 |
| 71 | HSPD1 | heat shock protein family D (Hsp60) member 1 | ENSG00000144381 | 3329 | NM_002156.5; NM_199440.2 |
| 72 | GZMA | granzyme A | ENSG00000145649 | 3001 | NM_006144.4 |
| 73 | JAK1 | Janus kinase 1 | ENSG00000162434 | 3716 | NM_002227.4; NM_001320923.2; NM_001321852.2; NM_001321853.2; NM_001321854.2; NM_001321855.2; NM_001321856.2; NM_001321857.2 |
| 74 | CHST12 | carbohydrate sulfotransferase 12 | ENSG00000136213 | 55501 | NM_001243794.2; NM_001243795.2; NM_018641.5 |
| 75 | ADAM19 | ADAM metallopeptidase domain 19 | ENSG00000135074 | 8728 | NM_033274.5 |
| 76 | PRF1 | perforin 1 | ENSG00000180644 | 5551 | NM_005041.6; NM_001083116.3 |
| 77 | CD63 | CD63 molecule | ENSG00000135404 | 967 | NM_001780.6; NM_001257389.2; NM_001257390.2; NM_001257391.2; NM_001257392.1; NM_001257400.2; NM_001257401.2; NM_001267698.2; NM_001413281.1; NM_001413282.1; NM_001413283.1; NM_001413284.1; NM_001413285.1; NM_001413286.1; NM_001413287.1; NM_001413288.1 |
| 78 | TM2D3 | TM2 domain containing 3 | ENSG00000184277 | 80213 | NM_078474.3; NM_025141.4; NM_001307960.2; NM_001308026.2 |
| 79 | CASP8 | caspase 8 | ENSG00000064012 | 841 | NM_001228.5; NM_033355.4; NM_033356.4; NM_001080124.2; NM_001080125.2; NM_001372051.1; NM_001400642.1; NM_001400645.1; NM_001400648.1; NM_001400651.1; NM_001400653.1; NM_001400654.1; NM_001400655.1; NM_001400656.1; NM_001400657.1; NM_001400658.1; NM_001400659.1; NM_001400660.1; NM_001400661.1; NM_001400662.1; NM_001400663.1; N M 001400664.1; NM_001400665.1; NM_001400666.1; NM_001400667.1; NM_001400668.1; NM_001400669.1; NM_001400670.1; NM_001400671.1; NM_001400672.1; NM_001400673.1; NM_001400674.1; NM_001400675.1; NM_001400676.1; NM_001400677.1; NM_001400678.1; NM_001400679.1; NM_001400680.1; NM_001400750.1; NM_001400751.1 |
| 80 | CARS1 | Cysteinyl-TRNA Synthetase 1 | ENSG00000110619 | 833 | NM_139273.4; NM_001751.6; NM_001014437.3; NM_001194997.2; NM_001378136.1; NM_001378137.1; NM_001378138.1; NM_001378139.1; NM_001378140.1 |
| 81 | ACP5 | acid phosphatase 5; tartrate resistant | ENSG00000102575 | 54 | NM_001111035.3; NM_001111034.3; NM_001111036.3; NM_001611.5; NM_001322023.2 |
| 82 | PTPN7 | protein tyrosine phosphatase non-receptor type 7 | ENSG00000143851 | 5778 | NM_002832.4; NM_080588.3; N M_001199797.2; N M _ 001364877.2; NM_001364878.1 |
| 83 | RHOH | ras homolog family member H | ENSG00000168421 | 399 | NM_001278359.2; NM_001278360.2; NM_001278361.2; NM_001278362.2; NM_001278363.2; NM_004310.5; NM_001278364.2; NM_001278365.2; NM_001278366.2; NM_001278367.2; NM_001278368.2; NM_001278369.2 |
| 84 | HAPLN3 | hyaluronan and proteoglycan link protein 3 | ENSG00000140511 | 145864 | NM_001307952.2; NM_178232.4 |
| 85 | CSTB | cystatin B | ENSG00000160213 | 1476 | NM_000100.4 |
| 86 | TLE5 | TLE family member 5; transcriptional modulator | ENSG00000104964 | 166 | NM_198969.1; NM_001130.6; NM_198970.2 |
| 87 | ACTG2 | actin gamma 2; smooth muscle | ENSG00000163017 | 72 | NM_001615.4; NM_001199893.2 |
| 88 | CD7 | CD7 molecule | ENSG00000173762 | 924 | NM_006137.7 |
| 89 | IL2RA | interleukin 2 receptor subunit alpha | ENSG00000134460 | 3559 | NM_000417.3; NM_001308242.2; NM_001308243.2 |
| 90 | CD99 | CD99 molecule (Xg blood group) | ENSG00000002586 | 4267 | NM_002414.5; NM_001122898.3; NM_001321367.2; NM_001321368.2; NM_001321369.2; NM_001321370.2 |
| 91 | CARHSP1 | calcium regulated heat stable protein 1 | ENSG00000153048 | 23589 | NM_014316.4; NM_001042476.2; NM_001278260.2; NM_001278261.2; NM_001278262.2; NM_001278263.1; NM_001278264.2; NM_001278265.2; NM_001278266.2 |
| 92 | TMSB4X | thymosin beta 4 X-linked | ENSG00000205542 | 7114 | NM_021109.4 |
| 93 | PSMB9 | proteasome 20S subunit beta 9 | ENSG00000240065 | 5698 | NM_002800.5 |
| 94 | CCR4 | C-C motif chemokine receptor 4 | ENSG00000183813 | 1233 | NM_005508.5 |
| 95 | AKAP13 | A-kinase anchoring protein 13 | ENSG00000170776 | 11214 | NM_006738.6; NM_007200.5; NM_001270546.1 |
| 96 | RABAC1 | Rab acceptor 1 | ENSG00000105404 | 10567 | NM_006423.3 |
| 97 | HLA-DRA | major histocompatibility complex; class II; DR alpha | ENSG00000204287 | 3122 | NM_019111.5 |
| 98 | FTH1 | ferritin heavy chain 1 | ENSG00000167996 | 2495 | NM_002032.3 |
| 99 | CD27 | CD27 molecule | ENSG00000139193 | 939 | NM_001413263.1; NM_001242.5; NM_001413264.1; NM_001413265.1; NM_001413266.1; NM_001413267.1; NM_001413268.1 |
| 100 | PCM1 | pericentriolar material 1 | ENSG00000078674 | 5108 | NM_006197.4; NM_001315507.2; NM_001315508.2; NM_001352632.2; NM_001352633.2; NM_001352634.2; NM_001352635.2; NM_001352636.2; NM_001352637.2; NM_001352638.2; NM_001352639.2; NM_001352640.2; NM_001352641.2; NM_001352642.2; NM_001352643.2; NM_001352644.2; NM_001352645.2; NM_001352646.2; NM_001352647.2; NM_001352648.2; NM_001352649.2; NM_001352650.2; NM_001352651.2; NM_001352652.2; NM_001352653.2; NM_001352654.2; NM_001352655.2; NM_001352656.2; |
| | | | | | NM_001352657.2; NM_001352658.2; NM_001352659.2; NM_001352660.2 |

**Table 2: Performance of predicTCR against gene signature approach on predicting BT21 tumour reactive TCR**

| **Methods** | **Threshold** | **TP** | **FP** | **TN** | **FN** | **Accuracy** | **G-Mean** | **AUC** |
|---|---|---|---|---|---|---|---|---|
| **predicTCR** | 0.52 | 12 | 1 | 8 | 1 | 0.91 | 0.91 | 0.92 |
| **NeoTCR8** | 0.39 | 2 | 0 | 9 | 11 | 0.5 | 0.15 | 0.87 |
| **Hanada** | 0.43 | 13 | 5 | 4 | 0 | 0.82 | 0.67 | 0.72 |
| **Caushi** | 0.46 | 13 | 5 | 4 | 0 | 0.77 | 0.67 | 0.72 |
| **Meng TR30** | 0.37 | 12 | 4 | 5 | 1 | 0.77 | 0.72 | 0.85 |

**Table 3: Performance of predicTCR in diverse tumor entities. Threshold = Fisher-Jenk break for calling reactive TCR. TP = True Positive, FP = False Positive, TN = True Negative, FN = False Negative**

| **Patients** | **Source** | **Type** | **Validation** | **Tech** | **Threshold** | **TP** | **FP** | **TN** | **FN** | **Accuracy** | **G-Mean** | **AUC** |
|---|---|---|---|---|---|---|---|---|---|---|---|---|
| TIPC249 | Meng et al. | PDAC | Cell Line | 10X | 0.39 | 5 | 0 | 0 | 1 | 0.83 | NA | NA |
| TIPC262 | Meng et al. | PDAC | Cell Line | 10X | 0.39 | 9 | 2 | 6 | 0 | 0.88 | 0.87 | 0.89 |
| TIPC282 | Meng et al. | PDAC | Cell Line | 10X | 0.39 | 8 | 1 | 4 | 0 | 0.92 | 0.89 | 0.98 |
| TIPC301 | Meng et al. | PDAC | Cell Line | 10X | 0.39 | 0 | 1 | 11 | 3 | 0.73 | 0.00 | 0.61 |
| TIPC309 | Meng et al. | PDAC | Cell Line | 10X | 0.39 | 21 | 0 | 13 | 0 | 1.00 | 1.00 | 1.00 |
| TIPC413 | Meng et al. | PDAC | PDX | 10X | 0.39 | 1 | 0 | 3 | 1 | 0.80 | 0.71 | 0.67 |
| TIPC416 | Meng et al. | PDAC | Cell Line | 10X | 0.39 | 3 | 1 | 2 | 2 | 0.63 | 0.63 | 0.53 |
| TIPC418 | Meng et al. | PDAC | Cell Line | 10X | 0.39 | 0 | 1 | 7 | 0 | 0.88 | NA | NA |
| TIPC432 | Meng et al. | PDAC | Cell Line | 10X | 0.39 | 8 | 0 | 3 | 1 | 0.92 | 0.94 | 0.89 |
| TIPC Overall | Meng et al. | PDAC | Cell Line | 10X | NA | 55 | 6 | 49 | 8 | 0.88 | 0.88 | 0.88 |

| SR4323 | Lowery et al. | Colon-Met | TMG | 10X | 0.45 | 11 | 3 | 4 | 0 | 0.83 | 0.76 | 0.96 |
|---|---|---|---|---|---|---|---|---|---|---|---|---|
| MD01-004 | Caushi et al. | NSCLC | Peptide | 10X | 0.57 | 8 | 2 | 4 | 0 | 0.86 | 0.82 | 1.00 |
| MD01-005 | Caushi et al. | NSCLC | Peptide | 10X | 0.45 | 1 | 0 | 12 | 2 | 0.87 | 0.58 | 0.89 |
| MD043-011 | Caushi et al. | NSCLC | Peptide | 10X | 0.54 | 2 | 0 | 0 | 0 | 1.00 | NA | NA |
| MD Overall | Caushi et al. | NSCLC | Peptide | 10X | NA | 11 | 2 | 16 | 2 | 0.87 | 0.87 | 0.94 |
| CRI3061 | Zheng et al. | GI (PDAC) | TMG + Pep | SS2* | 0.16 | 2 | 0 | 4 | 0 | 1.00 | 1.00 | 1.00 |
| CRI3244 | Zheng et al. | GI (PDAC) | TMG + Pep | SS2* | 0.24 | 0 | 1 | 7 | 0 | 0.88 | NA | NA |
| CRI3281 | Zheng et al. | GI (Bile Duct) | TMG + Pep | SS2 | 0.15 | 0 | 2 | 2 | 0 | 0.50 | NA | NA |
| CRI3395 | Zheng et al. | GI (Bile Duct) | TMG + Pep | SS2* | 0.15 | 1 | 1 | 5 | 0 | 0.86 | 0.91 | 1.00 |
| CRI3571 | Zheng et al. | GI (Bile Duct) | TMG + Pep | SS2* | 0.15 | 1 | 14 | 5 | 0 | 0.30 | 0.51 | 0.74 |
| CRI Overall | Zheng et al. | GI | TMG + Pep | SS2 | NA | 4 | 18 | 23 | 0 | 0.60 | 0.78 | 0.74 |

**Table 4: Performance of NeoTCR8 in diverse tumor entities. Threshold = Fisher-Jenk break for calling reactive TCR. TP = True Positive, FP = False Positive, TN = True Negative, FN = False Negative**

| **Patients** | **Source** | **Type** | **Validation** | **Tech** | **Threshold** | **TP** | **FP** | **TN** | **FN** | **Accuracy** | **G-Mean** | **AUC** |
|---|---|---|---|---|---|---|---|---|---|---|---|---|
| TIPC249 | Meng et al. | PDAC | Cell Line | 10X | 0.44 | 0 | 0 | 0 | 6 | 0.00 | NA | NA |
| TIPC262 | Meng et al. | PDAC | Cell Line | 10X | 0.44 | 1 | 0 | 8 | 8 | 0.53 | 0.33 | 0.60 |
| TIPC282 | Meng et al. | PDAC | Cell Line | 10X | 0.44 | 0 | 0 | 5 | 8 | 0.38 | 0.00 | 0.45 |
| TIPC301 | Meng et al. | PDAC | Cell Line | 10X | 0.44 | 0 | 0 | 12 | 3 | 0.80 | 0.00 | 0.42 |
| TIPC309 | Meng et al. | PDAC | Cell Line | 10X | 0.44 | 0 | 0 | 13 | 21 | 0.38 | 0.00 | 0.95 |
| TIPC413 | Meng et al. | PDAC | PDX | 10X | 0.44 | 0 | 0 | 3 | 2 | 0.60 | 0.00 | 0.33 |
| TIPC416 | Meng et al. | PDAC | Cell Line | 10X | 0.44 | 0 | 1 | 2 | 5 | 0.25 | 0.00 | 0.73 |
| TIPC418 | Meng et al. | PDAC | Cell Line | 10X | 0.44 | 0 | 1 | 7 | 0 | 0.88 | NA | NA |
| TIPC432 | Meng et al. | PDAC | Cell Line | 10X | 0.44 | 1 | 0 | 3 | 8 | 0.33 | 0.33 | 0.56 |
| TIPC Overall | Meng et al. | PDAC | Cell Line | 10X | NA | 2 | 2 | 53 | 61 | 0.47 | 0.03 | 0.65 |

| SR4323 | Lowery et al. | Colon-Met | TMG | 10X | 0.33 | 11 | 0 | 7 | 0 | 1.00 | 1.00 | 1.00 |
|---|---|---|---|---|---|---|---|---|---|---|---|---|
| MD01-004 | Caushi et al. | NSCLC | Peptide | 10X | NA* | 0 | 0 | 6 | 8 | 0.43 | 0.00 | 0.50 |
| MD01-005 | Caushi et al. | NSCLC | Peptide | 10X | 0.33 | 0 | 0 | 12 | 3 | 0.80 | 0.00 | 0.50 |
| MD043-011 | Caushi et al. | NSCLC | Peptide | 10X | NA* | 0 | 0 | 0 | 2 | 0.00 | NA | NA |
| MD Overall | Caushi et al. | NSCLC | Peptide | 10X | NA | 0 | 0 | 18 | 13 | 0.58 | 0.00 | 0.50 |
| CRI3061 | Zheng et al. | GI (PDAC) | TMG + Pep | SS2* | NA* | 0 | 0 | 4 | 2 | 0.67 | 0.00 | 0.50 |
| CRI3244 | Zheng et al. | GI (PDAC) | TMG + Pep | SS2* | NA* | 0 | 0 | 8 | 0 | 1.00 | NA | NA |
| CRI3281 | Zheng et al. | GI (Bile Duct) | TMG + Pep | SS2 | NA* | 0 | 0 | 4 | 0 | 1.00 | NA | NA |
| CRI3395 | Zheng et al. | GI (Bile Duct) | TMG + Pep | SS2* | NA* | 0 | 0 | 6 | 1 | 0.86 | 0.00 | 0.50 |
| CRI3571 | Zheng et al. | GI (Bile Duct) | TMG + Pep | SS2* | NA* | 0 | 0 | 19 | 1 | 0.95 | 0.00 | 0.50 |
| CRI Overall | Zheng et al. | GI | TMG + Pep | SS2 | NA | 0 | 0 | 41 | 4 | 0.91 | 0.00 | 0.50 |

**Table 5: Performance of Hanada et al. gene signature in diverse tumor entities. Threshold = Fisher-Jenk break for calling reactive TCR. TP = True Positive, FP = False Positive, TN = True Negative, FN = False Negative**

| **Patients** | **Source** | **Type** | **Validation** | **Tech** | **Threshold** | **TP** | **FP** | **TN** | **FN** | **Accuracy** | **G-Mean** | **AUC** |
|---|---|---|---|---|---|---|---|---|---|---|---|---|
| TIPC249 | Meng et al. | PDAC | Cell Line | 10X | 0.45 | 5 | 0 | 0 | 1 | 0.83 | NA | NA |
| TIPC262 | Meng et al. | PDAC | Cell Line | 10X | 0.45 | 9 | 5 | 3 | 0 | 0.71 | 0.61 | 0.69 |
| TIPC282 | Meng et al. | PDAC | Cell Line | 10X | 0.45 | 8 | 3 | 2 | 0 | 0.77 | 0.63 | 0.70 |
| TIPC301 | Meng et al. | PDAC | Cell Line | 10X | 0.45 | 0 | 4 | 8 | 3 | 0.53 | 0.00 | 0.33 |
| TIPC309 | Meng et al. | PDAC | Cell Line | 10X | 0.45 | 21 | 0 | 13 | 0 | 1.00 | 1.00 | 1.00 |
| TIPC413 | Meng et al. | PDAC | PDX | 10X | 0.45 | 2 | 1 | 2 | 0 | 0.80 | 0.82 | 0.83 |
| TIPC416 | Meng et al. | PDAC | Cell Line | 10X | 0.45 | 2 | 1 | 2 | 3 | 0.50 | 0.52 | 0.53 |
| TIPC418 | Meng et al. | PDAC | Cell Line | 10X | 0.45 | 0 | 5 | 3 | 0 | 0.38 | NA | NA |
| TIPC432 | Meng et al. | PDAC | Cell Line | 10X | 0.45 | 8 | 0 | 3 | 1 | 0.92 | 0.94 | 0.94 |
| TIPC Overall | Meng et al. | PDAC | Cell Line | 10X | NA | 55 | 19 | 36 | 8 | 0.77 | 0.76 | 0.76 |

| SR4323 | Lowery et al. | Colon-Met | TMG | 10X | 0.33 | 11 | 5 | 2 | 0 | 0.72 | 0.53 | 0.64 |
|---|---|---|---|---|---|---|---|---|---|---|---|---|
| MD01-004 | Caushi et al. | NSCLC | Peptide | 10X | 0.33 | 5 | 0 | 6 | 3 | 0.79 | 0.79 | 0.81 |
| MD01-005 | Caushi et al. | NSCLC | Peptide | 10X | 0.42 | 1 | 1 | 11 | 2 | 0.80 | 0.55 | 0.93 |
| MD043-011 | Caushi et al. | NSCLC | Peptide | 10X | 0.45 | 2 | 0 | 0 | 0 | 1.00 | NA | NA |
| MD Overall | Caushi et al. | NSCLC | Peptide | 10X | NA | 8 | 1 | 17 | 5 | 0.81 | 0.76 | 0.86 |
| CRI3061 | Zheng et al. | GI (PDAC) | TMG + Pep | SS2* | 0.43 | 2 | 0 | 4 | 0 | 1.00 | 1.00 | 1.00 |
| CRI3244 | Zheng et al. | GI (PDAC) | TMG + Pep | SS2* | 0.56 | 0 | 7 | 1 | 0 | 0.13 | NA | NA |
| CRI3281 | Zheng et al. | GI (Bile Duct) | TMG + Pep | SS2 | 0.50 | 0 | 4 | 0 | 0 | 0.00 | NA | NA |
| CRI3395 | Zheng et al. | GI (Bile Duct) | TMG + Pep | SS2* | NA** | 1 | 6 | 0 | 0 | 0.14 | 0.00 | 0.67 |
| CRI3571 | Zheng et al. | GI (Bile Duct) | TMG + Pep | SS2* | 0.50 | 1 | 17 | 2 | 0 | 0.15 | 0.32 | 0.84 |
| CRI Overall | Zheng et al. | GI | TMG + Pep | SS2 | NA | 4 | 34 | 7 | 0 | 0.24 | 0.41 | 0.54 |

**Table 6: Performance of Caushi et al. gene signature in diverse tumor entities. Threshold = Fisher-Jenk break for calling reactive TCR. TP = True Positive, FP = False Positive, TN = True Negative, FN = False Negative**

| **Patients** | **Source** | **Type** | **Validation** | **Tech** | **Threshold** | **TP** | **FP** | **TN** | **FN** | **Accuracy** | **G-Mean** | **AUC** |
|---|---|---|---|---|---|---|---|---|---|---|---|---|
| TIPC249 | Meng et al. | PDAC | Cell Line | 10X | 0.50 | 6 | 0 | 0 | 0 | 1.00 | NA | NA |
| TIPC262 | Meng et al. | PDAC | Cell Line | 10X | 0.50 | 9 | 8 | 0 | 0 | 0.53 | 0.00 | 0.50 |
| TIPC282 | Meng et al. | PDAC | Cell Line | 10X | 0.50 | 8 | 5 | 0 | 0 | 0.62 | 0.00 | 0.50 |
| TIPC301 | Meng et al. | PDAC | Cell Line | 10X | 0.50 | 3 | 12 | 0 | 0 | 0.20 | 0.00 | 0.50 |
| TIPC309 | Meng et al. | PDAC | Cell Line | 10X | 0.50 | 21 | 13 | 0 | 0 | 0.00 | 0.00 | 0.50 |
| TIPC413 | Meng et al. | PDAC | PDX | 10X | 0.50 | 2 | 2 | 1 | 0 | 0.60 | 0.58 | 0.67 |
| TIPC416 | Meng et al. | PDAC | Cell Line | 10X | 0.50 | 5 | 3 | 0 | 0 | 0.63 | 0.00 | 0.50 |
| TIPC418 | Meng et al. | PDAC | Cell Line | 10X | 0.50 | 0 | 8 | 0 | 0 | 0.00 | NA | NA |
| TIPC432 | Meng et al. | PDAC | Cell Line | 10X | 0.50 | 9 | 3 | 0 | 0 | 0.75 | 0.00 | 0.50 |
| TIPC Overall | Meng et al. | PDAC | Cell Line | 10X | NA | 63 | 54 | 1 | 0 | 0.54 | 0.13 | 0.51 |
| SR4323 | Lowery et al. | Colon-Met | TMG | 10X | 0.50 | 11 | 7 | 0 | 0 | 0.61 | 0.00 | 0.50 |
| MD01-004 | Caushi et al. | NSCLC | Peptide | 10X | 0.41 | 8 | 5 | 1 | 0 | 0.64 | 0.41 | 0.67 |
| MD01-005 | Caushi et al. | NSCLC | Peptide | 10X | 0.53 | 0 | 0 | 12 | 3 | 0.80 | 0.00 | 0.97 |
| MD043-011 | Caushi et al. | NSCLC | Peptide | 10X | 0.50 | 2 | 0 | 0 | 0 | 1.00 | NA | NA |
| MD Overall | Caushi et al. | NSCLC | Peptide | 10X | NA | 10 | 5 | 13 | 3 | 0.74 | 0.75 | 0.83 |
| CRI3061 | Zheng et al. | GI (PDAC) | TMG + Pep | SS2* | 0.50 | 2 | 4 | 0 | 0 | 0.33 | 0.00 | 0.63 |
| CRI3244 | Zheng et al. | GI (PDAC) | TMG + Pep | SS2* | 0.50 | 0 | 8 | 0 | 0 | 0.00 | NA | NA |
| CRI3281 | Zheng et al. | GI (Bile Duct) | TMG + Pep | SS2 | NA** | 0 | 4 | 0 | 0 | 0.00 | NA | NA |
| CRI3395 | Zheng et al. | GI (Bile Duct) | TMG + Pep | SS2* | 0.58 | 1 | 6 | 0 | 0 | 0.14 | 0.00 | 0.50 |
| CRI3571 | Zheng et al. | GI (Bile Duct) | TMG + Pep | SS2* | 0.75 | 1 | 18 | 1 | 0 | 0.10 | 0.23 | 0.53 |
| CRI Overall | Zheng et al. | GI | TMG + Pep | SS2 | NA | 4 | 40 | 1 | 0 | 0.11 | 0.16 | 0.57 |

**Table 7: Performance of TR30 gene signature in diverse tumor entities. Threshold = Fisher-Jenk break for calling reactive TCR. TP = True Positive, FP = False Positive, TN = True Negative, FN = False Negative**

| **Patients** | **Source** | **Type** | **Validation** | **Tech** | **Threshold** | **TP** | **FP** | **TN** | **FN** | **Accuracy** | **G-Mean** | **AUC** |
|---|---|---|---|---|---|---|---|---|---|---|---|---|
| TIPC249 | Meng et al. | PDAC | Cell Line | 10X | 0.47 | 5 | 0 | 0 | 1 | 0.83 | NA | NA |
| TIPC262 | Meng et al. | PDAC | Cell Line | 10X | 0.47 | 8 | 0 | 8 | 1 | 0.94 | 0.94 | 0.99 |
| TIPC282 | Meng et al. | PDAC | Cell Line | 10X | 0.47 | 2 | 0 | 5 | 6 | 0.54 | 0.50 | 0.93 |
| TIPC301 | Meng et al. | PDAC | Cell Line | 10X | 0.47 | 0 | 1 | 11 | 3 | 0.73 | 0.00 | 0.53 |
| TIPC309 | Meng et al. | PDAC | Cell Line | 10X | 0.47 | 21 | 0 | 13 | 0 | 1.00 | 1.00 | 1.00 |
| TIPC413 | Meng et al. | PDAC | PDX | 10X | 0.47 | 1 | 0 | 3 | 1 | 0.80 | 0.71 | 1.00 |
| TIPC416 | Meng et al. | PDAC | Cell Line | 10X | 0.47 | 1 | 1 | 2 | 4 | 0.38 | 0.37 | 0.47 |
| TIPC418 | Meng et al. | PDAC | Cell Line | 10X | 0.47 | 0 | 3 | 5 | 0 | 0.63 | NA | NA |
| TIPC432 | Meng et al. | PDAC | Cell Line | 10X | 0.47 | 7 | 0 | 3 | 2 | 0.83 | 0.88 | 0.89 |
| TIPC Overall | Meng et al. | PDAC | Cell Line | 10X | NA | 45 | 5 | 50 | 18 | 0.81 | 0.81 | 0.88 |
| SR4323 | Lowery et al. | Colon-Met | TMG | 10X | 0.22 | 11 | 5 | 2 | 0 | 0.72 | 0.53 | 0.84 |
| MD01-004 | Caushi et al. | NSCLC | Peptide | 10X | 0.27 | 8 | 3 | 3 | 0 | 0.79 | 0.71 | 0.94 |
| MD01-005 | Caushi et al. | NSCLC | Peptide | 10X | 0.23 | 3 | 4 | 8 | 0 | 0.73 | 0.82 | 1.00 |
| MD043-011 | Caushi et al. | NSCLC | Peptide | 10X | 0.33 | 2 | 0 | 0 | 0 | 1.00 | NA | NA |
| MD Overall | Caushi et al. | NSCLC | Peptide | 10X | NA | 13 | 7 | 11 | 0 | 0.77 | 0.78 | 0.98 |
| CRI3061 | Zheng et al. | GI (PDAC) | TMG + Pep | SS2* | 0.20 | 2 | 2 | 2 | 0 | 0.67 | 0.71 | 1.00 |
| CRI3244 | Zheng et al. | GI (PDAC) | TMG + Pep | SS2* | 0.28 | 0 | 7 | 1 | 0 | 0.13 | NA | NA |
| CRI3281 | Zheng et al. | GI (Bile Duct) | TMG + Pep | SS2 | 0.28 | 0 | 3 | 1 | 0 | 0.25 | NA | NA |
| CRI3395 | Zheng et al. | GI (Bile Duct) | TMG + Pep | SS2* | 0.28 | 1 | 5 | 1 | 0 | 0.29 | 0.41 | 0.83 |
| CRI3571 | Zheng et al. | GI (Bile Duct) | TMG + Pep | SS2* | 0.29 | 1 | 17 | 2 | 0 | 0.15 | 0.32 | 0.63 |
| CRI Overall | Zheng et al. | GI | TMG + Pep | SS2 | NA | 4 | 34 | 7 | 0 | 0.24 | 0.41 | 0.52 |

## Claims

1. A method of identifying a T cell reactive to target cells of a subject presenting a T cell activating epitope (reactive T cell), comprising
(a) determining expression of at least one biomarker selected from the group consisting of MAGEH1, HMGB2, TMSB10, MGAT4A, C12orf57, GIMAP7, HSPE1, STMN1, IL32, TPT1, MIF, PALM2-AKAP2, ISCU, CCR8, UGP2, EEF1B2, SETD7, MESD, LINC02099, IFT52, AKIRIN2, RIOK3, ANKRD12, CALR, DDX5, CNST, GNPDA1, MALAT1, RGL4, B2M, IFITM2, PTPN11, BTG1, ITSN2, RAD21, FYB1, DDX21, HSPD1, JAK1, ADAM19, TM2D3, CASP8, CARS1, PTPN7, HAPLN3, CSTB, TLE5, ACTG2, IL2RA, CD99, TMSB4X, CCR4, and PCM1 in T cells from a sample of said subject; and
(b) identifying a reactive T cell based on the determination of step (a).

2. The method of claim 1, wherein step (a) further comprises determining expression of at least one biomarker selected from the group consisting of ABI3, AC243829.4, ACP5, AKAP13, ANXA1, APOBEC3G, AREG, CARHSP1, CCL3, CCNH, CD27, CD63, CD7, CHST12, CLIC3, CRIP1, CST7, CTLA4, CTSC, CXCL13, FOXP3, FTH1, GALNT2, GZMA, HLA-DRA, HMOX1, IFNG, IKZF3, IL6ST, IL7R, KIR2DL4, KLF6, LINC02446, LTB, LYAR, MIR4435-2HG, NKG7, PKM, PRF1, PSMB9, RABAC1, RBPJ, RGS1, RHOH, RSRP1, SPOCK2, and TOX.

3. The method of claim 1 or 2, wherein at least 10, preferably at least 20, more preferably at least 30, even more preferably at least 38, most preferably at least 48, of said biomarkers are determined.

4. A method of identifying a TCR binding to an activating epitope presented on a cell, preferably a cancer cell, of a subject, said method comprising
(A) identifying a reactive T cell according to the method of any one of claims 1 to 3,
(B) providing the amino acid sequences of at least the complementarity determining regions (CDRs) 3 of the TCR of the reactive T cell identified in step (A); and, hereby,
(C) identifying a TCR binding to an activating epitope presented on a cell.

5. The method of claim 4, wherein at least one biomarker of step (A) and/or the nucleic acid sequences in step (B) is/are determined by single-cell sequencing, preferably by single-cell RNA sequencing.

6. The method of claim 4 or 5, wherein said method comprises further step (B1) expressing a TCR comprising at least the CDRs determined in step (B) in a host cell, preferably a T cell.

7. The method of claim 6, wherein said method comprises further step (B2) determining binding of the TCR expressed in step (B1) to an activating epitope presented on a cell, preferably a cancer epitope, complexed in a major histocompatibility complex (MHC), preferably MHC class I, molecule.

8. A method of providing a T cell recognizing a cell presenting a T cell activating epitope, preferably a cancer cell, said method comprising
(i) identifying a TCR binding to a cell presenting a T cell activating epitope according to the method according to any one of claims 4 to 7,
(ii) expressing a TCR comprising at least the complementarity determining regions (CDRs) of the TCR of step (i) in a T cell, and, thereby,
(iii) providing a T cell recognizing a cell presenting a T cell activating epitope, preferably a cancer cell.

9. A reactive T cell identified by the method according to any one of claims 1 to 3 and/or obtained or obtainable by the method according to claim 8.

10. A polynucleotide encoding at least one TCR binding to an activating epitope provided or identified according to the method according to any one claims 4 to 7.

11. A computer-implemented method of training at least one trainable model for identifying a T cell reactive to target cells of a subject presenting a T cell activating epitope (reactive T cell), the method comprising:
(A) providing the trainable model;
(B) retrieving allocated training data, the allocated training data comprising gene expression data of T cells allocated to reactivities of the T cell receptors (TCRs) of said T cells to said target cells, wherein said gene expression data and said reactivities of the TCRs were obtained by the method according to any one of claims 1 to 3; and
(C) training the trainable model on the allocated training data.

12. The method of claim 11, wherein the trainable model is selected from a trainable XGBoost model, a trainable Adaboost model, a trainable Decision Tree model, a trainable Catboost model, a trainable Gaussian Naive Bayes model, a trainable K-nearest neighbour model, a trainable Logistic regression model, a trainable Radial Basis Function (RBF) Support Vector Machine (SVM) model, a trainable Random Forest model, and a trainable linear SVM model

13. The method of claim 11 or 12, wherein the gene expression data of the allocated training data in step (B) are derived from tumor-infiltrating lymphocytes and are, preferably, obtained by mRNA analysis.

14. The method of any one of claims 11 to 13, wherein said method comprises a step of feature selection based on Shapley additive explanation (SHAP) values and repetition of hyperparameter tuning using the selected features.

15. A computer-implemented method of predicting reactivity of a T cell receptor to target cells of a subject, the method comprising
(I) retrieving at least one trained model according to the method according to any one of claims 11 to 14;
(II) retrieving gene expression data from T cells of said subject, the gene expression data comprising at least one predetermined target set of relevant genes; and
(III) applying the trained model to the gene expression data of step (II), thereby predicting reactivities of T cell receptors (TCRs) of said T cells to said target cells.
